# EUROPEAN PATENT APPLICATION

(11) **EP 3 791 930 A1**
(43) Date of publication of application: **17.03.2021**
(21) Application number: 19197308.0
(22) Date of filing: 13.09.2019
(51) Int. Cl.: A61P 35/00, A61K 31/7125, C12N 15/113

(54) **INHIBITOR OF METADHERIN EXPRESSION**

(71) Applicant: Secarna Pharmaceuticals GmbH & Co. KG, 35037 Marburg (DE); Firebrand Therapeutics, Inc., Princeton Junction, NJ 08550 (US)
(72) Inventor: Jaschinski, Frank, 35037 Marburg (DE); Klar, Richard, 35037 Marburg (DE); Michel, Sven, 35037 Marburg (DE); Jin, John Fenyu, Princeton Junction, NJ 08550 (US)
(74) Representative: V.O.

(57) **Abstract**

The present invention refers to an inhibitor consisting of an oligonucleotide comprising 12 to 25 nucleotides, wherein at least one of the nucleotides is modified, and the oligonucleotide hybridizes with a nucleic acid sequence of MTDH of SEQ ID NO.1 (human mRNA), SEQ ID NO.2 (human pre-mRNA), SEQ ID NO.223 (mouse mRNA) and/or SEQ ID NO.224 (mouse pre-mRNA), wherein the oligonucleotide inhibits at least 50 % of the MTDH expression. The invention is further directed to a pharmaceutical composition comprising such oligonucleotide.

## Description

The present disclosure refers to an inhibitor of metadherin (MTDH) expression consisting of an antisense oligonucleotide hybridizing with a nucleic acid sequence of MTDH and to a pharmaceutical composition comprising such antisense oligonucleotide and a pharmaceutically acceptable carrier, excipient and/or dilutant. Further, the invention refers to the use of the inhibitor or the pharmaceutical composition comprising the inhibitor in a method of preventing and/or treating a benign or malign tumor.

### Technical background

Metadherin (MTDH) also known as protein LYRIC or astrocyte elevated gene-1 protein (AEG-1) is for example involved in HIF-1alpha mediated angiogenesis. MTDH also interacts with SND1 and is involved in the RNA-induced silencing complex (RISC) and plays very important role in RISC and miRNA functions. It induces an oncogene called Late SV40 factor (LSF/TFCP2) which is involved in thymidylate synthase (TS) induction and DNA biosynthesis synthesis. Late SV40 factor (LSF/TFCP2) enhances angiogenesis by transcriptionally up-regulating matrix metalloproteinase-9 (MMP9). MTDH also regulates multiple signaling pathways including PI3K/Akt, NF-κB, Wnt/6-catenin, and MAPK which cooperate to promote the tumorigenic and metastatic potential of transformed cells. Several microRNA have also been found to be associated with the increased MTDH expression in different cancers.

MTDH is a type-two transmembrane protein containing an extracellular lung homing domain which is for example implicated in breast cancer metastasis to the lung. MTDH encodes a single-pass transmembrane protein with the molecular mass of 64-kDa expressed mainly in the endoplasmic reticulum and perinuclear space. In polarized epithelial cells, it co-localizes with tight junction protein ZO-1 and occludin; however, MTDH is not a native component of tight junctions but becomes incorporated during tight junction complex maturation. The sub-cellular location of MTDH protein varies depending on the physiological state of the cell. In non-malignant tissue, MTDH was shown to be expressed in the nucleus, whereas in malignant cells it becomes translocated into the cytoplasm. It is believed that cytoplasmic translocation of MTDH promotes disease progression by mediating mechanisms that support pro-angiogenesic and metastatic pathways.

It appears that TNF-α is the key regulator of MTDH expression. TNF-α upregulates MTDH expression via NF-κB pathways. TNF-α causes NF-κB nuclear translocation and consequent interaction with MTDH, which is essential for activation of downstream genes. The N-terminal domain of MTDH interacts with NF-κB and triggers gene expression via several convergent mechanisms. NF-κB nuclear translocation coincides with a significant reduction of IκBα level, suggesting MTDH involvement in IκBα degradation. Studies have also revealed that MTDH interacts with Cyclic AMP-responsive element binding protein-binding protein (CBP) which is a NF-κB coactivator. Hence, MTDH may function as a bridging element among p50-p65, NF-κB CBP, and the basal transcription machinery and therefore consequent induction of NF-κB related gene expression enhances migration and invasion. MTDH promoted NF-κB gene expression results in anchorage independent cell growth, possibly mediated by direct activation of matrix metalloproteinase 1 (MMP1) expression. MTDH also serves as a link between NF-κB and matrix metalloprotease 9 (MMP9) expression (Dhiman G. et al., Front. Oncol., 3 May 2019, Vol. 9, p. 1-8).

MTDH has recently been identified as being overexpressed in different cancers and is correlated with worse prognosis (Wan L. et al., Cancer Res 74, 2014, p. 5336-5347; Hu G. et al., Cancer Cell 15, 2009, p. 9-20; Song et al., J Pathol 219, 2009, p. 317-326). It has been identified as a factor that is responsible for cancer cell growth, progression and metastasis as shown for example in models of breast cancer (Wan L et al., Cancer Cell 26, 2014, p. 92-105) and prostate cancer (Wan L. et al., Cancer Res 74, 2014, p. 5336-5347). The proliferative effects of MTDH are related to attenuation of the key cell cycle inhibitors p27Kip1 and p21Cip1. MTDH furthermore mediates chemoresistance of cancer cells by increasing cell survival, probably by inhibiting pro-apoptotic genes like BNIP3 and TRAIL.

As MTDH is an intracellular factor with no enzymatic function it cannot directly be inhibited by monoclonal antibodies and specific suppression of its diverse functions with small molecule inhibitors is nearly impossible. It therefore represents an ideal target for antisense oligonucleotides.

So far no antisense oligonucleotide exists which is highly efficient in reduction and inhibition, respectively, of MTDH expression and hybridizes with MTDH mRNA and/or pre-mRNA.

An oligonucleotide of the present invention is very successful in the inhibition of the expression of MTDH. The mode of action of an oligonucleotide differs from the mode of action of an antibody or small molecule, and oligonucleotides are highly advantageous regarding for example
(i) the penetration of tumor tissue in solid tumors,
(ii) the blocking of multiple functions and activities, respectively, of a target,
(iii) the combination of oligonucleotides with each other or an antibody or a small molecule, and
(iv) the inhibition of intracellular effects which are not accessible for an antibody or inhibitable via a small molecule.

### Summary

The present invention refers to a MTDH inhibitor consisting of an antisense oligonucleotide comprising 12 to 25 nucleotides, wherein at least one of the nucleotides is modified, and the oligonucleotide hybridizes with a nucleic acid sequence of MTDH of SEQ ID NO.1, of SEQ ID NO.2 or a combination thereof, wherein the oligonucleotide inhibits at least 50 % of the MTDH expression.

The modified nucleotide is for example selected from the group consisting of a bridged nucleic acid such as LNA, cET, ENA, 2'Fluoro modified nucleotide, 2'O-Methyl modified nucleotide, a 2'O-Methoxy modified nucleotide, a FANA and a combination thereof.

The inhibitor and antisense oligonucleotide, respectively, of the present invention hybridizes for example with a hybridizing active region selected from the group consisting of position 52000 to 52499, position 32000 to 32499, position 87500 to 87999, position 90500 to 90999, position 65500 to 65999, position 8500 to 8999, position 9000 to 9499, position 9500 to 9999, position 1000 to 10499, position 10500 to 10999, position 11000 to 11499, position 13000 to 13499, position 14000 to 14499, position 15500 to 15999, position 16500 to 16999, position 17500 to 17999, position 18000 to 18499, position 20500 to 20999, position 21000 to 21499, position 22500 to 22999, position 24000 to 24499, position 25000 to 25499, position 25500 to 25999, position 27000 to 27499, position 29000 to 29499, position 29500 to 29999, position 37000 to 37499, position 37500 to 37999, position 43500 to 43999, position 44500 to 44999, position 45500 to 45999, position 46500 to 46999, position 47000 to 47499, position 49000 to 49499, position 50500 to 50999, position 52000 to 52499, position 54000 to 54499, position 55500 to 55999, position 61500 to 61999, position 64000 to 64499, position 64500 to 64999, position 65000 to 65499, position 68000 to 68499, position 68500 to 68999, position 71500 to 71999, position 72000 to 72499, position 74500 to 74999, position 76000 to 76499, position 77000 to 77499, position 77500 to 77999, position 78000 to 78499, position 80500 to 80999, position 81000 to 81499, position 81500 to 81999, position 82000 to 82499, position 83500 to 83999, position 85000 to 85499, position 86000 to 86499, position 88500 to 88999, position 89000 to 89499, position 89500 to 89999, position 90000 to 90499, position 91000 to 91499, position 92000 to 92499, position 92500 to 92999, position 93500 to 93999, position 94500 to 94999 of SEQ ID NO.2 or a combination thereof.

The inhibitor and antisense oligonucleotide, respectively, of the present invention comprises for example a sequence selected from the group consisting of SEQ ID NO. 13, SEQ ID NO.64, SEQ ID NO.20, SEQ ID NO.21, SEQ ID NO.29, SEQ ID NO.27, SEQ ID NO.79, one of SEQ ID NO.3 to SEQ ID NO.12, one of SEQ ID NO.14 to SEQ ID NO.19, one of SEQ ID NO.22 to SEQ ID NO.26, SEQ ID NO.28, one of SEQ ID NO.30 to SEQ ID NO.63, one of SEQ ID NO.65 to SEQ ID NO.78, one of SEQ ID NO.80 to SEQ ID NO.221 and a combination thereof.

The inhibitor and antisense oligonucleotide, respectively, of the present invention, wherein the antisense oligonucleotide is for example selected from the group consisting of +G*+T*+A*A*G*T*T*G*C*T*C*G*G*T*+G*+G*+T (A34011HM; SEQ ID NO.13),
+C*+A*+C*G*G*C*T*T*G*T*C*T*A*T*+C*+A*+G (A34062Hi; SEQ ID NO.64),
+T*+T*+G*T*A*G*T*A*T*T*G*G*C*+G*+G*+C (A34018H; SEQ ID NO.20),
+C*+T*+T*G*T*A*G*T*A*T*T*G*G*C*+G*+G*+C (A34019H; SEQ ID NO.21),
+C*+G*+C*A*A*T*A*C*T*G*T*T*G*A*+A*+C*+C (A34027H; SEQ ID NO.29),
+C*+G*+T*T*T*G*G*T*A*A*A*G*G*C*+T*+A*+T (A34025HM; SEQ ID NO.27),
+T*+C*+G*T*A*T*C*T*A*C*T*G*T*C*+T*+A*+A (A34077Hi; SEQ ID NO.79),
+C*+T*+T*A*T*C*A*C*G*T*T*T*A*C*+G*+C*+T (A34010H; SEQ ID NO.12),
+G*+A*+T*G*C*G*G*T*T*G*T*A*A*G*+T*+T*+G (A34012H; SEQ ID NO.14),
+T*+G*+C*T*C*G*G*T*G*G*T*A*A*C*+T*+G*+T (A34113HM; SEQ ID NO.115),
+A*+A*+G*T*T*G*C*T*C*G*G*T*G*G*+T*+A*+A (A34114HM; SEQ ID NO.116),
+T*+G*+A*T*G*C*G*G*T*T*G*T*A*A*+G*+T*+T (A34115H; SEQ ID NO.117),
+A*+A*+C*A*C*T*G*C*T*G*G*T*A*T*+T*+C*+G (A34137Hi; SEQ ID NO. 139),
+A*+G*+C*T*T*C*C*T*T*T*A*A*G*C*+G*+A*+C (A34063Hi; SEQ ID NO.65),
+C*+G*+T*T*C*T*T*G*G*C*G*C*C*A*+C*+A*+T (A34026H; SEQ ID NO.28),
+C*+A*+C*G*T*T*T*G*G*T*A*A*A*G*+G*+C*+T (A34122HM; SEQ ID NO. 124),
+C*+G*+C*C*A*G*C*T*T*A*C*C*T*T*+G*+A*+T (A34075Hi; SEQ ID NO.77),
+T*+G*+T*C*G*C*C*A*G*C*T*T*A*C*+C*+T*+T (A34189Hi; SEQ ID NO.191) and a combination thereof, wherein + indicates an LNA nucleotide and * indicates a phosphorothioate (PTO) linkage between the nucleotides.

The inhibitor of the present invention inhibits for example the expression of MTDH at a nanomolar or micromolar concentration.

The present invention further refers to a pharmaceutical composition comprising an inhibitor of the present invention and a pharmaceutically acceptable carrier, excipient, dilutant or a combination thereof. Optionally, the pharmaceutical composition further comprises another active agent, another oligonucleotide, an antibody, a peptide-based therapeutic, a protein-based therapeutic and/or a small molecule.

The Inhibitor and the pharmaceutical composition, respectively, of the present invention are for example for use in a method of preventing and/or treating a disorder, where an MTDH imbalance is involved. The disorder is for example a tumor such as a malignant or benign tumor, which is for example selected from the group consisting of breast cancer, lung cancer, malignant melanoma, lymphoma, skin cancer, bone cancer, prostate cancer, liver cancer, brain cancer, cancer of the larynx, gall bladder, pancreas, testicular, rectum, parathyroid, thyroid, adrenal, neural tissue, head and neck, colon, stomach, bronchi, kidneys, basal cell carcinoma, squamous cell carcinoma, metastatic skin carcinoma, osteo sarcoma, Ewing's sarcoma, reticulum cell sarcoma, liposarcoma, myeloma, giant cell tumor, small-cell lung tumor, islet cell tumor, primary brain tumor, meningioma, acute and chronic lymphocytic and granulocytic tumors, acute and chronic myeloid leukemia, hairy-cell tumor, adenoma, hyperplasia, medullary carcinoma, intestinal ganglioneuromas, Wilm's tumor, seminoma, ovarian tumor, leiomyomater tumor, cervical dysplasia, retinoblastoma, soft tissue sarcoma, malignant carcinoid, topical skin lesion, rhabdomyosarcoma, Kaposi's sarcoma, osteogenic sarcoma, malignant hypercalcemia, renal cell tumor, polycythermia vera, adenocarcinoma, anaplastic astrocytoma, glioblastoma multiforma, leukemia, epidermoid carcinoma and a kidney disease such as diabetic nephropathy.

The inhibitor or the pharmaceutical composition of the present invention is for example suitable to be administered locally or systemically.

All documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference.

### Description of figures

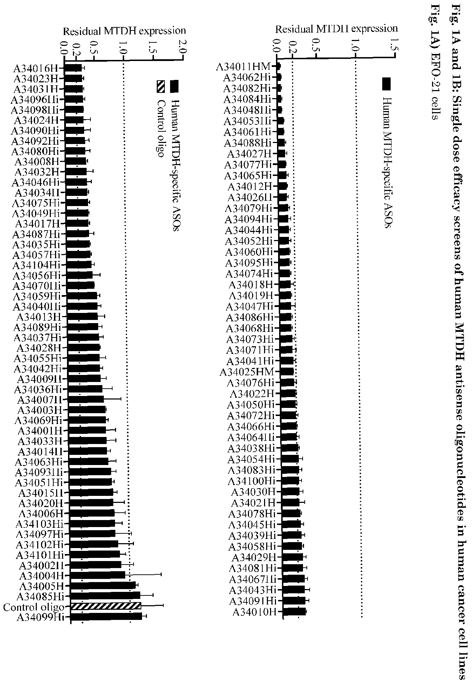

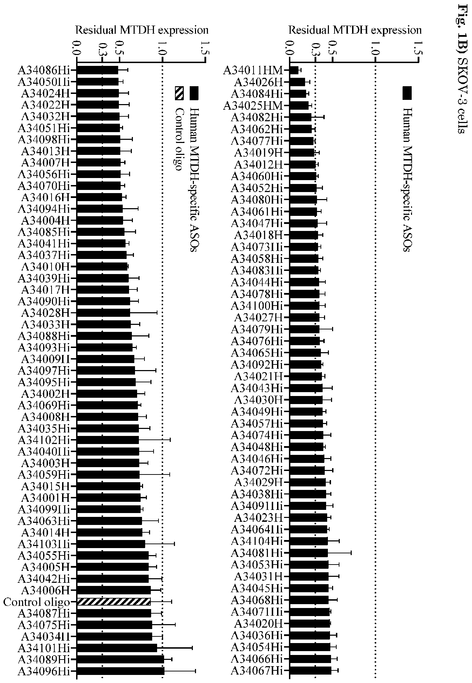

**Fig. 1A** and **Fig. 1B** show efficacy screening experiments EFO-21 (**Fig. 1A**) and SKOV-3 (**Fig. 1B**). The cells were treated with the respective human specific antisense oligonucleotide at a concentration of 5 µM for three days without the use of a transfection reagent. MTDH expression values are normalized to HPRT1 values and residual MTDH expression as compared to mock-treated cells (set to 1) is depicted in **Fig. 1A** and **Fig. 1B****.**

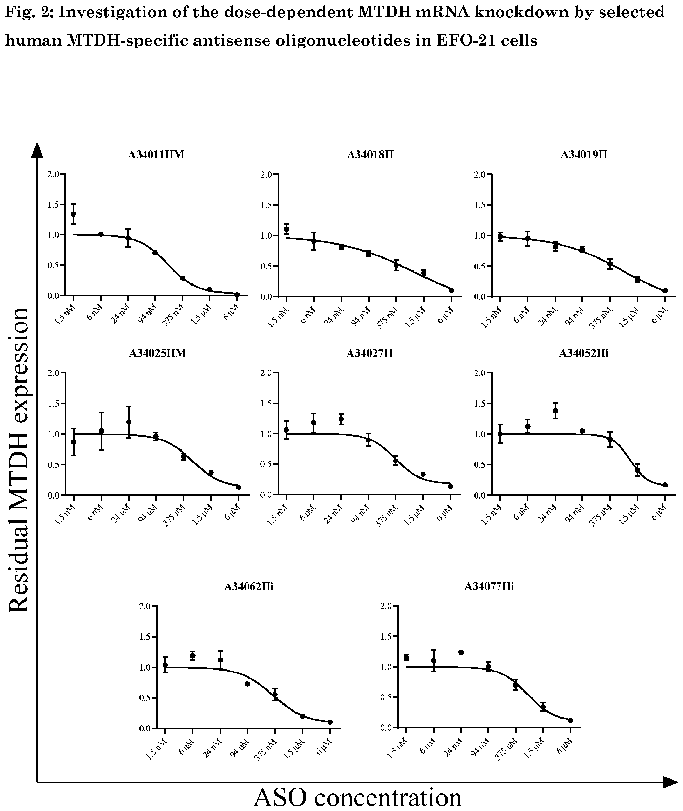

**Fig. 2** shows the dose-dependent knockdown of MTDH mRNA expression by human specific MTDH antisense oligonucleotides in EFO-21 cells. The cells were treated for three days with the respective antisense oligonucleotide at the following concentrations without the use of a transfection reagent: 6 µM, 1.5 µM, 375 nM, 94 nM, 24 nM, 6 nM, 1.5 nM. The MTDH expression values are normalized to HPRT1 values and residual MTDH expression as compared to mock-treated cells (set to 1) is depicted.

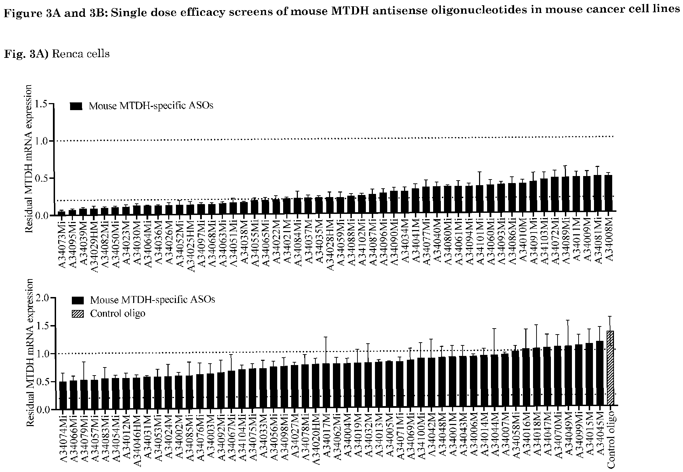

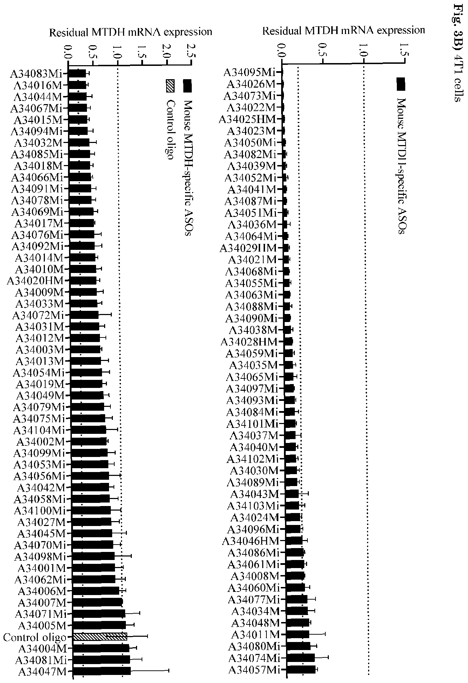

**Fig. 3A** and **Fig. 3B** depicts efficacy screening experiments Renca (**Fig. 3A**) and 4T1 (**Fig. 3B**). The cells were treated with the respective mouse specific antisense oligonucleotide at a concentration of 5 µM for three days without the use of a transfection reagent. MTDH expression values are normalized to HPRT1 values and residual MTDH expression as compared to mock-treated cells (set to 1) is depicted in **Fig. 3A** and **Fig. 3B****.**

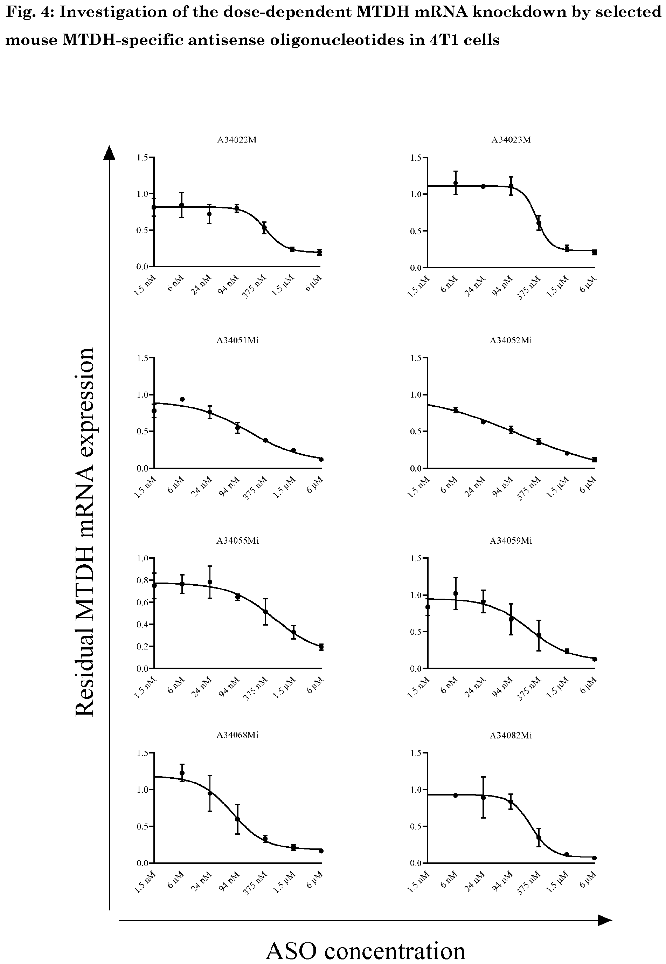

**Fig. 4** shows the dose-dependent knockdown of MTDH mRNA expression by mouse specific MTDH antisense oligonucleotides in 4T1 cells. The cells were treated for three days with the respective antisense oligonucleotide at the following concentrations without the use of a transfection reagent: 6 µM, 1.5 µM, 375 nM, 94 nM, 24 nM, 6 nM, 1.5 nM. The MTDH expression values are normalized to HPRT1 values.

### Detailed description

The present invention provides for the first time human and murine antisense oligonucleotides which hybridize with mRNA and pre-mRNA sequences of MTDH and inhibit the expression and activity, respectively, of MTDH. Thus, the oligonucleotides of the present invention represent an interesting and highly efficient tool for use in a method of preventing and/or treating disorders, where the MTDH expression and activity, respectively, is increased in comparison to a healthy subject. The MTDH expression for example is involved in the induction of the disease and/or mediates resistance to another therapy. The oligonucleotide of the present invention hybridizes for example with a nucleic acid sequence of MTDH of SEQ ID NO.1 (human mRNA), SEQ ID NO.2 (human pre-mRNA), SEQ ID NO.223 (mouse mRNA) and/or SEQ ID NO.224 (mouse pre-mRNA), wherein the oligonucleotide inhibits at least 50 % of the MTDH expression.

In the following, the elements of the present invention will be described in more detail. These elements are listed with specific embodiments, however, it should be understood that they may be combined in any manner and in any number to create additional embodiments. The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise.

Throughout this specification and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example"), provided herein is intended merely to better illustrate the invention and does not pose a limitation on the scope of the invention otherwise claimed. No language in the specification should be construed as indicating any non-claimed element essential to the practice of the invention.

An oligonucleotide of the present invention is for example an antisense oligonucleotide (ASO) consisting of or comprising 10 to 25 nucleotides, 10 to 15 nucleotides, 15 to 20 nucleotides, 12 to 18 nucleotides, or 15 to 17 nucleotides. The oligonucleotides for example consist of or comprise 10, 11, 12, 13, 14, 15, 16, 17, 18, 19, 20, 21, 22, 23, 24 or 25 nucleotides. The oligonucleotides of the present invention comprise at least one nucleotide which is modified. The modified nucleotide is for example a bridged nucleotide such as a locked nucleic acid (LNA, e.g., 2',4'-LNA), cET, ENA, a 2'Fluoro modified nucleotide, a 2'O-Methyl modified nucleotide or a combination thereof. In some embodiments, the oligonucleotide of the present invention comprises nucleotides having the same or different modifications. In some embodiments the oligonucleotide of the present invention comprises a modified phosphate backbone, wherein the phosphate is for example a phosphorothioate.

The oligonucleotide of the present invention comprises the one or more modified nucleotide at the 3'- and/or 5'- end of the oligonucleotide and/or at any position within the oligonucleotide, wherein modified nucleotides follow in a row of 1, 2, 3, 4, 5, or 6 modified nucleotides, or a modified nucleotide is combined with one or more unmodified nucleotides. The following Table 1 presents embodiments of oligonucleotides comprising modified nucleotides for example LNA which are indicated by (+) and phosphorothioate (PTO) indicated by (*). The oligonucleotides consisting of or comprising the sequences of Table 1 may comprise any other modified nucleotide and/or any other combination of modified and unmodified nucleotides. Oligonucleotides of Table 1 hybridize with exonic regions of the mRNA of human MTDH (SEQ ID NO.1; NM_178812.3) or with intronic regions of the pre-mRNA of human MTDH (SEQ ID NO.2; chr8:976353340-97738900), indicated by "i" in the following Table 1:

**Table 1: List of antisense oligonucleotides hybridizing with human MTDH mRNA for example of SEQ ID 1 and / or intronic regions of the human MTDH pre-mRNA (Hi) for example of SEQ ID 2. Some antisense oligonucleotides also hybridize with mouse MTDH mRNA (HM). The antisense oligonucleotides were designed according to in house criteria and negl (described in WO2014154843 A1) was used as a control oligonucleotide in all experiments.**

| Seq ID | Name | Antisense Sequence 5'-3' | Antisense Sequence 5'-3' with PTO (*) and LNA (+) |
|---|---|---|---|
| 3 | A34001H | GAACAATGGCGGCTTG | +G*+A*+A*C*A*A*T*G*G*C*G*G*C*+T*+T*+G |
| 4 | A34002H | TCGGCGGAACAATGGC | +T*+C*+G*G*C*G*G*A*A*C*A*A*T*+G*+G*+C |
| 5 | A34003H | CTCGGCGGAACAATGGC | +C*+T*+C*G*G*C*G*G*A*A*C*A*A*T*+G*+G*+C |
| 6 | A34004H | ACGCAGTGGAATAGTCG | +A*+C*+G*C*A*G*T*G*G*A*A*T*A*G*+T*+C*+G |
| 7 | A34005H | AGGCCGACCGAGAGCAT | +A*+G*+G*C*C*G*A*C*C*G*A*G*A*G*+C*+A*+T |
| 8 | A34006H | AGCTCGGTGCGCAGAAA | +A*+G*+C*T*C*G*G*T*G*C*G*C*A*G*+A*+A*+A |
| 9 | A34007H | GTACCGTTTCGGCTCCA | +G*+T*+A*C*C*G*T*T*T*C*G*G*C*T*+C*+C*+A |
| 10 | A34008H | TCGCTCCGGAGATTCTT | +T*+C*+G*C*T*C*C*G*G*A*G*A*T*T*+C*+T*+T |
| 11 | A34009H | GCCACTTCAACAGTCCG | +G*+C*+C*A*C*T*T*C*A*A*C*A*G*T*+C*+C*+G |
| 12 | A34010H | CTTATCACGTTTACGCT | +C*+T*+T*A*T*C*A*C*G*T*T*T*A*C*+G*+C*+T |
| 13 | A34011HM | GTAAGTTGCTCGGTGGT | +G*+T*+A*A*G*T*T*G*C*T*C*G*G*T*+G*+G*+T |
| 14 | A34012H | GATGCGGTTGTAAGTTG | +G*+A*+T*G*C*G*G*T*T*G*T*A*A*G*+T*+T*+G |
| 15 | A34013H | GAACGGTCACTCCAACT | +G*+A*+A*C*G*G*T*C*A*C*T*C*C*A*+A*+C*+T |
| 16 | A34014H | CTTCGGCTGGATCACTA | +C*+T*+T*C*G*G*C*T*G*G*A*T*C*A*+C*+T*+A |
| 17 | A34015H | GTACTTCGGCTGGATCA | +G*+T*+A*C*T*T*C*G*G*C*T*G*G*A*+T*+C*+A |
| 18 | A34016H | ACGAGTACTTCGGCTGG | +A*+C*+G*A*G*T*A*C*T*T*C*G*G*C*+T*+G*+G |
| 19 | A34017H | TTTGACGAGTACTTCGG | +T*+T*+T*G*A*C*G*A*G*T*A*C*T*T*+C*+G*+G |
| 20 | A34018H | TTGTAGTATTGGCGGC | +T*+T*+G*T*A*G*T*A*T*T*G*G*C*+G*+G*+C |
| 21 | A34019H | CTTGTAGTATTGGCGGC | +C*+T*+T*G*T*A*G*T*A*T*T*G*G*C*+G*+G*+C |
| 22 | A34020H | ACGTTTCTCGTCTGGCT | +A*+C*+G*T*T*T*C*T*C*G*T*C*T*G*+G*+C*+T |
| 23 | A34021H | TCACGTTTCTCGTCTGG | +T*+C*+A*C*G*T*T*T*C*T*C*G*T*C*+T*+G*+G |
| 24 | A34022H | GATCGTTCTTGTTCTAT | +G*+A*+T*C*G*T*T*C*T*T*G*T*T*C*+T*+A*+T |
| 25 | A34023H | TACGCACTACAGGTTAA | +T*+A*+C*G*C*A*C*T*A*C*A*G*G*T*+T*+A*+A |
| 26 | A34024H | CATATTCTACGCACTAC | +C*+A*+T*A*T*T*C*T*A*C*G*C*A*C*+T*+A*+C |
| 27 | A34025HM | CGTTTGGTAAAGGCTAT | +C*+G*+T*T*T*G*G*T*A*A*A*G*G*C*+T*+A*+T |
| 28 | A34026H | CGTTCTTGGCGCCACAT | +C*+G*+T*T*C*T*T*G*G*C*G*C*C*A*+C*+A*+T |
| 29 | A34027H | CGCAATACTGTTGAACC | +C*+G*+C*A*A*T*A*C*T*G*T*T*G*A*+A*+C*+C |
| 30 | A34028H | ACCTCAACTTAGCAACG | +A*+C*+C*T*C*A*A*C*T*T*A*G*C*A*+A*+C*+G |
| 31 | A34029H | TCGGTACAGATAGGTAG | +T*+C*+G*G*T*A*C*A*G*A*T*A*G*G*+T*+A*+G |
| 32 | A34030H | TCAATTGTCGGTACAGA | +T*+C*+A*A*T*T*G*T*C*G*G*T*A*C*+A*+G*+A |
| 33 | A34031H | TTGCTCAATTGTCGGTA | +T*+T*+G*C*T*C*A*A*T*T*G*T*C*G*+G*+T*+A |
| 34 | A34032H | TGCCGATAGGAAGTTTC | +T*+G*+C*C*G*A*T*A*G*G*A*A*G*T*+T*+T*+C |
| 35 | A34033H | GCTCAGATGCCGATAGG | +G*+C*+T*C*A*G*A*T*G*C*C*G*A*T*+A*+G*+G |
| 36 | A34034H | CATCGTCCTGTTAGAGT | +C*+A*+T*C*G*T*C*C*T*G*T*T*A*G*+A*+G*+T |
| 37 | A34035Hi | CCGTTCTCTACTGCCGC | +C*+C*+G*T*T*C*T*C*T*A*C*T*G*C*+C*+G*+C |
| 38 | A34036Hi | CTCGGCTTTCGACTAAG | +C*+T*+C*G*G*C*T*T*T*C*G*A*C*T*+A*+A*+G |
| 39 | A34037Hi | TCGTCCTATACTTCCTG | +T*+C*+G*T*C*C*T*A*T*A*C*T*T*C*+C*+T*+G |
| 40 | A34038Hi | TTATTCTAGCGGTGACG | +T*+T*+A*T*T*C*T*A*G*C*G*G*T*G*+A*+C*+G |
| 41 | A34039Hi | CTTCCGAGCGCAGTCTT | +C*+T*+T*C*C*G*A*G*C*G*C*A*G*T*+C*+T*+T |
| 42 | A34040Hi | CTAGGTCACCGCACTTC | +C*+T*+A*G*G*T*C*A*C*C*G*C*A*C*+T*+T*+C |
| 43 | A34041Hi | GAAGCGCGTCTAGACCT | +G*+A*+A*G*C*G*C*G*T*C*T*A*G*A*+C*+C*+T |
| 44 | A34042Hi | CGTTCCGGCCTCTGTTG | +C*+G*+T*T*C*C*G*G*C*C*T*C*T*G*+T*+T*+G |
| 45 | A34043Hi | CGTATTAGGTAACCGAC | +C*+G*+T*A*T*T*A*G*G*T*A*A*C*C*+G*+A*+C |
| 46 | A34044Hi | CTCGTTCGTTGAACTCG | +C*+T*+C*G*T*T*C*G*T*T*G*A*A*C*+T*+C*+G |
| 47 | A34045Hi | CCAGTAAATCGGTGCCT | +C*+C*+A*G*T*A*A*A*T*C*G*G*T*G*+C*+C*+T |
| 48 | A34046Hi | AAGTGATACGCACTAGA | +A*+A*+G*T*G*A*T*A*C*G*C*A*C*T*+A*+G*+A |
| 49 | A34047Hi | GATCTTAATTCGCTGGA | +G*+A*+T*C*T*T*A*A*T*T*C*G*C*T*+G*+G*+A |
| 50 | A34048Hi | CTTCGTACTGGCTTACT | +C*+T*+T*C*G*T*A*C*T*G*G*C*T*T*+A*+C*+T |
| 51 | A34049Hi | TACGTGTATACCTTGCA | +T*+A*+C*G*T*G*T*A*T*A*C*C*T*T*+G*+C*+A |
| 52 | A34050Hi | AACCACTTACCGATCAG | +A*+A*+C*C*A*C*T*T*A*C*C*G*A*T*+C*+A*+G |
| 53 | A34051Hi | CGTGAAGGCCTATCCAG | +C*+G*+T*G*A*A*G*G*C*C*T*A*T*C*+C*+A*+G |
| 54 | A34052Hi | TTATAACGCATGTCGGA | +T*+T*+A*T*A*A*C*G*C*A*T*G*T*C*+G*+G*+A |
| 55 | A34053Hi | AAGCATTCGCCGGAATC | +A*+A*+G*C*A*T*T*C*G*C*C*G*G*A*+A*+T*+C |
| 56 | A34054Hi | TATAAGCATTCGCCGGA | +T*+A*+T*A*A*G*C*A*T*T*C*G*C*C*+G*+G*+A |
| 57 | A34055Hi | GCTAACGCTAGGTTTAC | +G*+C*+T*A*A*C*G*C*T*A*G*G*T*T*+T*+A*+C |
| 58 | A34056Hi | TTGCAATATAAGCGACT | +T*+T*+G*C*A*A*T*A*T*A*A*G*C*G*+A*+C*+T |
| 59 | A34057Hi | GGTCAGGCATAACACTC | +G*+G*+T*C*A*G*G*C*A*T*A*A*C*A*+C*+T*+C |
| 60 | A34058Hi | GAATAAGCTGCGTTCAC | +G*+A*+A*T*A*A*G*C*T*G*C*G*T*T*+C*+A*+C |
| 61 | A34059Hi | TATGAGCTATAACCGCC | +T*+A*+T*G*A*G*C*T*A*T*A*A*C*C*+G*+C*+C |
| 62 | A34060Hi | CCAAGATATGGCTCCGA | +C*+C*+A*A*G*A*T*A*T*G*G*C*T*C*+C*+G*+A |
| 63 | A34061Hi | GTGTGTGACCACTAGTA | +G*+T*+G*T*G*T*G*A*C*C*A*C*T*A*+G*+T*+A |
| 64 | A34062Hi | CACGGCTTGTCTATCAG | +C*+A*+C*G*G*C*T*T*G*T*C*T*A*T*+C*+A*+G |
| 65 | A34063Hi | AGCTTCCTTTAAGCGAC | +A*+G*+C*T*T*C*C*T*T*T*A*A*G*C*+G*+A*+C |
| 66 | A34064Hi | TTACTAGTGCGTTGAGA | +T*+T*+A*C*T*A*G*T*G*C*G*T*T*G*+A*+G*+A |
| 67 | A34065Hi | TTTACGTTAGGCCTCTG | +T*+T*+T*A*C*G*T*T*A*G*G*C*C*T*+C*+T*+G |
| 68 | A34066Hi | CAGACGCTGCGGAACTA | +C*+A*+G*A*C*G*C*T*G*C*G*G*A*A*+C*+T*+A |
| 69 | A34067Hi | CCAGATACGGTTCTCAC | +C*+C*+A*G*A*T*A*C*G*G*T*T*C*T*+C*+A*+C |
| 70 | A34068Hi | TGATCCATCGTCCAAGT | +T*+G*+A*T*C*C*A*T*C*G*T*C*C*A*+A*+G*+T |
| 71 | A34069Hi | GTATGCTTAATAGGCCG | +G*+T*+A*T*G*C*T*T*A*A*T*A*G*G*+C*+C*+G |
| 72 | A34070Hi | CATACAGCCGTGTCTAC | +C*+A*+T*A*C*A*G*C*C*G*T*G*T*C*+T*+A*+C |
| 73 | A34071Hi | CAGTAGTACCTTGTACG | +C*+A*+G*T*A*G*T*A*C*C*T*T*G*T*+A*+C*+G |
| 74 | A34072Hi | CTATACTTTGACACGGA | +C*+T*+A*T*A*C*T*T*T*G*A*C*A*C*+G*+G*+A |
| 75 | A34073Hi | GAGCTTTCCGAACATAC | +G*+A*+G*C*T*T*T*C*C*G*A*A*C*A*+T*+A*+C |
| 76 | A34074Hi | CACCGAACACCTATGTA | +C*+A*+C*C*G*A*A*C*A*C*C*T*A*T*+G*+T*+A |
| 77 | A34075Hi | CGCCAGCTTACCTTGAT | +C*+G*+C*C*A*G*C*T*T*A*C*C*T*T*+G*+A*+T |
| 78 | A34076Hi | GTGCTCATACGCTCCTA | +G*+T*+G*C*T*C*A*T*A*C*G*C*T*C*+C*+T*+A |
| 79 | A34077Hi | TCGTATCTACTGTCTAA | +T*+C*+G*T*A*T*C*T*A*C*T*G*T*C*+T*+A*+A |
| 80 | A34078Hi | GTACTCTAACCGTCTTT | +G*+T*+A*C*T*C*T*A*A*C*C*G*T*C*+T*+T*+T |
| 81 | A34079Hi | TTGTCTCCGAGCCTTAT | +T*+T*+G*T*C*T*C*C*G*A*G*C*C*T*+T*+A*+T |
| 82 | A34080Hi | GCCGTCCATAAGCCATG | +G*+C*+C*G*T*C*C*A*T*A*A*G*C*C*+A*+T*+G |
| 83 | A34081Hi | TAGTAGCACAGAGGCGA | +T*+A*+G*T*A*G*C*A*C*A*G*A*G*G*+C*+G*+A |
| 84 | A34082Hi | CGTGCAGCTTGTAGTCT | +C*+G*+T*G*C*A*G*C*T*T*G*T*A*G*+T*+C*+T |
| 85 | A34083Hi | TCTAACGTACAAACGCT | +T*+C*+T*A*A*C*G*T*A*C*A*A*A*C*+G*+C*+T |
| 86 | A34084Hi | ATGTACTCGTGCTCTGG | +A*+T*+G*T*A*C*T*C*G*T*G*C*T*C*+T*+G*+G |
| 87 | A34085Hi | CCGACCGATTGAGGCCA | +C*+C*+G*A*C*C*G*A*T*T*G*A*G*G*+C*+C*+A |
| 88 | A34086Hi | GATACCACGTGTTGCTA | +G*+A*+T*A*C*C*A*C*G*T*G*T*T*G*+C*+T*+A |
| 89 | A34087Hi | TACCGATGTTCAATGCC | +T*+A*+C*C*G*A*T*G*T*T*C*A*A*T*+G*+C*+C |
| 90 | A34088Hi | TTAGTTTATTCCGGATC | +T*+T*+A*G*T*T*T*A*T*T*C*C*G*G*+A*+T*+C |
| 91 | A34089Hi | TTTAGGAGCCGAGTATA | +T*+T*+T*A*G*G*A*G*C*C*G*A*G*T*+A*+T*+A |
| 92 | A34090Hi | GAGTCTGTTAACGACAA | +G*+A*+G*T*C*T*G*T*T*A*A*C*G*A*+C*+A*+A |
| 93 | A34091Hi | TACCATAGCCGCTCTTA | +T*+A*+C*C*A*T*A*G*C*C*G*C*T*C*+T*+T*+A |
| 94 | A34092Hi | GAATAGATACGTGCCAT | +G*+A*+A*T*A*G*A*T*A*C*G*T*G*C*+C*+A*+T |
| 95 | A34093Hi | GGTGGTCTCACGAACTA | +G*+G*+T*G*G*T*C*T*C*A*C*G*A*A*+C*+T*+A |
| 96 | A34094Hi | GATGATTCTAGTAACCG | +G*+A*+T*G*A*T*T*C*T*A*G*T*A*A*+C*+C*+G |
| 97 | A34095Hi | CGTGTATAAGTGGAGGT | +C*+G*+T*G*T*A*T*A*A*G*T*G*G*A*+G*+G*+T |
| 98 | A34096Hi | ACCTATACGCATACAAG | +A*+C*+C*T*A*T*A*C*G*C*A*T*A*C*+A*+A*+G |
| 99 | A34097Hi | CCAGCGATTGTACATAT | +C*+C*+A*G*C*G*A*T*T*G*T*A*C*A*+T*+A*+T |
| 100 | A34098Hi | GTGTCTACGTCATCCAT | +G*+T*+G*T*C*T*A*C*G*T*C*A*T*C*+C*+A*+T |
| 101 | A34099Hi | AGCCGGAGGCGCTTAAT | +A*+G*+C*C*G*G*A*G*G*C*G*C*T*T*+A*+A*+T |
| 102 | A34100Hi | CCTTTGCCAATACGTTA | +C*+C*+T*T*T*G*C*C*A*A*T*A*C*G*+T*+T*+A |
| 103 | A34101Hi | TTTGGTGCGGTAGCTTG | +T*+T*+T*G*G*T*G*C*G*G*T*A*G*C*+T*+T*+G |
| 104 | A34102Hi | CTAGGACACCATGGTAC | +C*+T*+A*G*G*A*C*A*C*C*A*T*G*G*+T*+A*+C |
| 105 | A34103Hi | ATTGCGGATTGCGTCAC | +A*+T*+T*G*C*G*G*A*T*T*G*C*G*T*+C*+A*+C |
| 106 | A34104Hi | ACAGATGTTCGGCCAGC | +A*+C*+A*G*A*T*G*T*T*C*G*G*C*C*+A*+G*+C |
| 107 | A34105H | GAACAATGGCGGCTTGG | +G*+A*+A*C*A*A*T*G*G*C*G*G*C*T*+T*+G*+G |
| 108 | A34106H | GGAACAATGGCGGCTTG | +G*+G*+A*A*C*A*A*T*G*G*C*G*G*C*+T*+T*+G |
| 109 | A34107H | CTCGGCGGAACAATGG | +C*+T*+C*G*G*C*G*G*A*A*C*A*A*+T*+G*+G |
| 110 | A34108H | CCTCGGCGGAACAATGG | +C*+C*+T*C*G*G*C*G*G*A*A*C*A*A*+T*+G*+G |
| 111 | A34109HM | TAGGCCGACCGAGAGCA | +T*+A*+G*G*C*C*G*A*C*C*G*A*G*A*+G*+C*+A |
| 112 | A34110HM | TAGGCCGACCGAGAGC | +T*+A*+G*G*C*C*G*A*C*C*G*A*G*+A*+G*+C |
| 113 | A34111H | TACCGTTTCGGCTCCAG | +T*+A*+C*C*G*T*T*T*C*G*G*C*T*C*+C*+A*+G |
| 114 | A34112H | CCACTTCAACAGTCCGC | +C*+C*+A*C*T*T*C*A*A*C*A*G*T*C*+C*+G*+C |
| 115 | A34113HM | TGCTCGGTGGTAACTGT | +T*+G*+C*T*C*G*G*T*G*G*T*A*A*C*+T*+G*+T |
| 116 | A34114HM | AAGTTGCTCGGTGGTAA | +A*+A*+G*T*T*G*C*T*C*G*G*T*G*G*+T*+A*+A |
| 117 | A34115H | TGATGCGGTTGTAAGTT | +T*+G*+A*T*G*C*G*G*T*T*G*T*A*A*+G*+T*+T |
| 118 | A34116HM | AGACCATTCATCATCGA | +A*+G*+A*C*C*A*T*T*C*A*T*C*A*T*+C*+G*+A |
| 119 | A34117H | ACGAGTACTTCGGCTG | +A*+C*+G*A*G*T*A*C*T*T*C*G*G*+C*+T*+G |
| 120 | A34118H | TGACGAGTACTTCGGCT | +T*+G*+A*C*G*A*G*T*A*C*T*T*C*G*+G*+C*+T |
| 121 | A34119H | GGCTATTTTTGACGAGT | +G*+G*+C*T*A*T*T*T*T*T*G*A*C*G*+A*+G*+T |
| 122 | A34120H | CACGTTTCTCGTCTGGC | +C*+A*+C*G*T*T*T*C*T*C*G*T*C*T*+G*+G*+C |
| 123 | A34121H | ACGCACTACAGGTTAAG | +A*+C*+G*C*A*C*T*A*C*A*G*G*T*T*+A*+A*+G |
| 124 | A34122HM | CACGTTTGGTAAAGGCT | +C*+A*+C*G*T*T*T*G*G*T*A*A*A*G*+G*+C*+T |
| 125 | A34123H | TTTGCTCAATTGTCGGT | +T*+T*+T*G*C*T*C*A*A*T*T*G*T*C*+G*+G*+T |
| 126 | A34124H | GATGCCGATAGGAAGTT | +G*+A*+T*G*C*C*G*A*T*A*G*G*A*A*+G*+T*+T |
| 127 | A34125H | TGGCTCAGATGCCGATA | +T*+G*+G*C*T*C*A*G*A*T*G*C*C*G*+A*+T*+A |
| 128 | A34126H | ATCGTCCTGTTAGAGTA | +A*+T*+C*G*T*C*C*T*G*T*T*A*G*A*+G*+T*+A |
| 129 | A34127Hi | CTCTCGGCTTTCGACTA | +C*+T*+C*T*C*G*G*C*T*T*T*C*G*A*+C*+T*+A |
| 130 | A34128Hi | CCTCTCGGCTTTCGACT | +C*+C*+T*C*T*C*G*G*C*T*T*T*C*G*+A*+C*+T |
| 131 | A34129Hi | CGTCCTATACTTCCTGA | +C*+G*+T*C*C*T*A*T*A*C*T*T*C*C*+T*+G*+A |
| 132 | A34130Hi | CTAGCGGTGACGGTTC | +C*+T*+A*G*C*G*G*T*G*A*C*G*G*+T*+T*+C |
| 133 | A34131Hi | GTGCTTATTCTAGCGGT | +G*+T*+G*C*T*T*A*T*T*C*T*A*G*C*+G*+G*+T |
| 134 | A34132Hi | AAGCGCGTCTAGACCT | +A*+A*+G*C*G*C*G*T*C*T*A*G*A*+C*+C*+T |
| 135 | A34133Hi | TCAAAGCGTATTAGGTA | +T*+C*+A*A*A*G*C*G*T*A*T*T*A*G*+G*+T*+A |
| 136 | A34134Hi | CACCTCAAAGCGTATTA | +C*+A*+C*C*T*C*A*A*A*G*C*G*T*A*+T*+T*+A |
| 137 | A34135Hi | ACACCTCAAAGCGTATT | +A*+C*+A*C*C*T*C*A*A*A*G*C*G*T*+A*+T*+T |
| 138 | A34136Hi | TCGTTGAACTCGCCTTA | +T*+C*+G*T*T*G*A*A*C*T*C*G*C*C*+T*+T*+A |
| 139 | A34137Hi | AACACTGCTGGTATTCG | +A*+A*+C*A*C*T*G*C*T*G*G*T*A*T*+T*+C*+G |
| 140 | A34138Hi | ACTCGTTCGTTGAACTC | +A*+C*+T*C*G*T*T*C*G*T*T*G*A*A*+C*+T*+C |
| 141 | A34139Hi | CTCGTTCGTTGAACTC | +C*+T*+C*G*T*T*C*G*T*T*G*A*A*+C*+T*+C |
| 142 | A34140Hi | CAGACTCGTTCGTTGAA | +C*+A*+G*A*C*T*C*G*T*T*C*G*T*T*+G*+A*+A |
| 143 | A34141Hi | ACAGACTCGTTCGTTGA | +A*+C*+A*G*A*C*T*C*G*T*T*C*G*T*+T*+G*+A |
| 144 | A34142Hi | ACAGACTCGTTCGTTG | +A*+C*+A*G*A*C*T*C*G*T*T*C*G*+T*+T*+G |
| 145 | A34143Hi | CACAAATCTACCACGCC | +C*+A*+C*A*A*A*T*C*T*A*C*C*A*C*+G*+C*+C |
| 146 | A34144Hi | GTAAATCGGTGCCTGAA | +G*+T*+A*A*A*T*C*G*G*T*G*C*C*T*+G*+A*+A |
| 147 | A34145Hi | AGTAAATCGGTGCCTGA | +A*+G*+T*A*A*A*T*C*G*G*T*G*C*C*+T*+G*+A |
| 148 | A34146Hi | CGTACTGGCTTACTGAA | +C*+G*+T*A*C*T*G*G*C*T*T*A*C*T*+G*+A*+A |
| 149 | A34147Hi | TTCGTACTGGCTTACTG | +T*+T*+C*G*T*A*C*T*G*G*C*T*T*A*+C*+T*+G |
| 150 | A34148Hi | TCTACGTGTATACCTTG | +T*+C*+T*A*C*G*T*G*T*A*T*A*C*C*+T*+T*+G |
| 151 | A34149Hi | CCACCAGTATTGGATAG | +C*+C*+A*C*C*A*G*T*A*T*T*G*G*A*+T*+A*+G |
| 152 | A34150Hi | CGCCGGAATCTGTATTC | +C*+G*+C*C*G*G*A*A*T*C*T*G*T*A*+T*+T*+C |
| 153 | A34151Hi | ATTCGCCGGAATCTGTA | +A*+T*+T*C*G*C*C*G*G*A*A*T*C*T*+G*+T*+A |
| 154 | A34152Hi | AGCATTCGCCGGAATC | +A*+G*+C*A*T*T*C*G*C*C*G*G*A*+A*+T*+C |
| 155 | A34153Hi | TAAGCATTCGCCGGAAT | *+T*+A*+A*G*C*A*T*T*C*G*C*C*G*G*+A*+A*+T |
| 156 | A34154Hi | AAGCATTCGCCGGAAT | +A*+A*+G*C*A*T*T*C*G*C*C*G*G*+A*+A*+T |
| 157 | A34155Hi | ATAAGCATTCGCCGGAA | +A*+T*+A*A*G*C*A*T*T*C*G*C*C*G*+G*+A*+A |
| 158 | A34156Hi | TAAGCATTCGCCGGAA | +T*+A*+A*G*C*A*T*T*C*G*C*C*G*+G*+A*+A |
| 159 | A34157Hi | ATAAGCATTCGCCGGA | +A*+T*+A*A*G*C*A*T*T*C*G*C*C*+G*+G*+A |
| 160 | A34158Hi | TGTATAAGCATTCGCCG | +T*+G*+T*A*T*A*A*G*C*A*T*T*C*G*+C*+C*+G |
| 161 | A34159Hi | GTATAAGCATTCGCCG | +G*+T*+A*T*A*A*G*C*A*T*T*C*G*+C*+C*+G |
| 162 | A34160Hi | ACGCTAGGTTTACTTAT | +A*+C*+G*C*T*A*G*G*T*T*T*A*C*T*+T*+A*+T |
| 163 | A34161Hi | AACGCTAGGTTTACTTA | +A*+A*+C*G*C*T*A*G*G*T*T*T*A*C*+T*+T*+A |
| 164 | A34162Hi | AGCTAACGCTAGGTTTA | +A*+G*+C*T*A*A*C*G*C*T*A*G*G*T*+T*+T*+A |
| 165 | A34163Hi | GCGTTCACATCCTAGTC | +G*+C*+G*T*T*C*A*C*A*T*C*C*T*A*+G*+T*+C |
| 166 | A34164Hi | AAGACTCTACTCACACG | +A*+A*+G*A*C*T*C*T*A*C*T*C*A*C*+A*+C*+G |
| 167 | A34165Hi | TACTAGTGCGTTGAGAA | +T*+A*+C*T*A*G*T*G*C*G*T*T*G*A*+G*+A*+A |
| 168 | A34166Hi | ATTACTAGTGCGTTGAG | +A*+T*+T*A*C*T*A*G*T*G*C*G*T*T*+G*+A*+G |
| 169 | A34167Hi | TCATTACTAGTGCGTTG | +T*+C*+A*T*T*A*C*T*A*G*T*G*C*G*+T*+T*+G |
| 170 | A34168Hi | GAACATGCCATCGAAAC | +G*+A*+A*C*A*T*G*C*C*A*T*C*G*A*+A*+A*+C |
| 171 | A34169Hi | GATACGGTTCTCACATT | +G*+A*+T*A*C*G*G*T*T*C*T*C*A*C*+A*+T*+T |
| 172 | A34170Hi | AGATACGGTTCTCACAT | +A*+G*+A*T*A*C*G*G*T*T*C*T*C*A*+C*+A*+T |
| 173 | A34171Hi | CAGATACGGTTCTCACA | +C*+A*+G*A*T*A*C*G*G*T*T*C*T*C*+A*+C*+A |
| 174 | A34172Hi | ACTTTCACTTAGTTACG | +A*+C*+T*T*T*C*A*C*T*T*A*G*T*T*+A*+C*+G |
| 175 | A34173Hi | TAGCACACGGCACAAGC | +T*+A*+G*C*A*C*A*C*G*G*C*A*C*A*+A*+G*+C |
| 176 | A34174Hi | ATAGCACACGGCACAAG | +A*+T*+A*G*C*A*C*A*C*G*G*C*A*C*+A*+A*+G |
| 177 | A34175Hi | ACATGATCCATCGTCCA | +A*+C*+A*T*G*A*T*C*C*A*T*C*G*T*+C*+C*+A |
| 178 | A34176Hi | ATACATGATCCATCGTC | +A*+T*+A*C*A*T*G*A*T*C*C*A*T*C*+G*+T*+C |
| 179 | A34177Hi | TAACTCTTATTCGGTCC | +T*+A*+A*C*T*C*T*T*A*T*T*C*G*G*+T*+C*+C |
| 180 | A34178Hi | CTAACTCTTATTCGGTC | +C*+T*+A*A*C*T*C*T*T*A*T*T*C*G*+G*+T*+C |
| 181 | A34179Hi | CAGCCGTGTCTACCTAA | +C*+A*+G*C*C*G*T*G*T*C*T*A*C*C*+T*+A*+A |
| 182 | A34180Hi | ACAGCCGTGTCTACCTA | +A*+C*+A*G*C*C*G*T*G*T*C*T*A*C*+C*+T*+A |
| 183 | A34181Hi | ACATACAGCCGTGTCTA | +A*+C*+A*T*A*C*A*G*C*C*G*T*G*T*+C*+T*+A |
| 184 | A34182Hi | GAATTACATACAGCCGT | +G*+A*+A*T*T*A*C*A*T*A*C*A*G*C*+C*+G*+T |
| 185 | A34183Hi | GTACGCAGAAGGTATTC | +G*+T*+A*C*G*C*A*G*A*A*G*G*T*A*+T*+T*+C |
| 186 | A34184Hi | GGCATGAGCTTTCCGAA | +G*+G*+C*A*T*G*A*G*C*T*T*T*C*C*+G*+A*+A |
| 187 | A34185Hi | TCATACGCTCCTATCTG | +T*+C*+A*T*A*C*G*C*T*C*C*T*A*T*+C*+T*+G |
| 188 | A34186Hi | TACGCTCCTATCTGTGC | +T*+A*+C*G*C*T*C*C*T*A*T*C*T*G*+T*+G*+C |
| 189 | A34187Hi | ATACGCTCCTATCTGTG | +A*+T*+A*C*G*C*T*C*C*T*A*T*C*T*+G*+T*+G |
| 190 | A34188Hi | CATACGCTCCTATCTGT | +C*+A*+T*A*C*G*C*T*C*C*T*A*T*C*+T*+G*+T |
| 191 | A34189Hi | TGTCGCCAGCTTACCTT | +T*+G*+T*C*G*C*C*A*G*C*T*T*A*C*+C*+T*+T |
| 192 | A34190Hi | GCTCATACGCTCCTATC | +G*+C*+T*C*A*T*A*C*G*C*T*C*C*T*+A*+T*+C |
| 193 | A34191Hi | AAGGTGCTCATACGCTC | +A*+A*+G*G*T*G*C*T*C*A*T*A*C*G*+C*+T*+C |
| 194 | A34192Hi | CCAAAGGTGCTCATACG | +C*+C*+A*A*A*G*G*T*G*C*T*C*A*T*+A*+C*+G |
| 195 | A34193Hi | GCCAAAGGTGCTCATAC | +G*+C*+C*A*A*A*G*G*T*G*C*T*C*A*+T*+A*+C |
| 196 | A34194Hi | CGAGCCTTATTTCTACA | +C*+G*+A*G*C*C*T*T*A*T*T*T*C*T*+A*+C*+A |
| 197 | A34195Hi | CAATGCAGTAAGCGCTC | +C*+A*+A*T*G*C*A*G*T*A*A*G*C*G*+C*+T*+C |
| 198 | A34196Hi | ACGTACAAACGCTCTTT | +A*+C*+G*T*A*C*A*A*A*C*G*C*T*C*+T*+T*+T |
| 199 | A34197Hi | TAACGTACAAACGCTCT | +T*+A*+A*C*G*T*A*C*A*A*A*C*G*C*+T*+C*+T |
| 200 | A34198Hi | CCTACCGATGTTCAATG | +C*+C*+T*A*C*C*G*A*T*G*T*T*C*A*+A*+T*+G |
| 201 | A34199Hi | GAGCCGAGTATACATAA | +G*+A*+G*C*C*G*A*G*T*A*T*A*C*A*+T*+A*+A |
| 202 | A34200Hi | TAGGAGCCGAGTATACA | +T*+A*+G*G*A*G*C*C*G*A*G*T*A*T*+A*+C*+A |
| 203 | A34201Hi | GTCTGTTAACGACAAAG | +G*+T*+C*T*G*T*T*A*A*C*G*A*C*A*+A*+A*+G |
| 204 | A34202Hi | AAGAGTCTGTTAACGAC | +A*+A*+G*A*G*T*C*T*G*T*T*A*A*C*+G*+A*+C |
| 205 | A34203Hi | TAGCCGCTCTTAAGTAA | +T*+A*+G*C*C*G*C*T*C*T*T*A*A*G*+T*+A*+A |
| 206 | A34204Hi | AATAGATACGTGCCATG | +A*+A*+T*A*G*A*T*A*C*G*T*G*C*C*+A*+T*+G |
| 207 | A34205Hi | TGGTGGTCTCACGAACT | +T*+G*+G*T*G*G*T*C*T*C*A*C*G*A*+A*+C*+T |
| 208 | A34206Hi | TTACCATAGCCGCTCTT | +T*+T*+A*C*C*A*T*A*G*C*C*G*C*T*+C*+T*+T |
| 209 | A34207Hi | GTCTTTACCATAGCCGC | +G*+T*+C*T*T*T*A*C*C*A*T*A*G*C*+C*+G*+C |
| 210 | A34208Hi | CCTATACGCATACAAGT | +C*+C*+T*A*T*A*C*G*C*A*T*A*C*A*+A*+G*+T |
| 211 | A34209Hi | GGAACTAGTATCTGTAC | +G*+G*+A*A*C*T*A*G*T*A*T*C*T*G*+T*+A*+C |
| 212 | A34210Hi | CAGTGTGTCTACGTCAT | +C*+A*+G*T*G*T*G*T*C*T*A*C*G*T*+C*+A*+T |
| 213 | A34211Hi | TCAGTGTGTCTACGTCA | +T*+C*+A*G*T*G*T*G*T*C*T*A*C*G*+T*+C*+A |
| 214 | A34212Hi | AAGCCGGAGGCGCTTAA | +A*+A*+G*C*C*G*G*A*G*G*C*G*C*T*+T*+A*+A |
| 215 | A34213Hi | AGCCGGAGGCGCTTAA | +A*+G*+C*C*G*G*A*G*G*C*G*C*T*+T*+A*+A |
| 216 | A34214Hi | CCAGCTACCACGTGCGG | +C*+C*+A*G*C*T*A*C*C*A*C*G*T*G*+C*+G*+G |
| 217 | A34215Hi | TTGCGGATTGCGTCACT | +T*+T*+G*C*G*G*A*T*T*G*C*G*T*C*+A*+C*+T |
| 218 | A34216Hi | TTGCGGATTGCGTCAC | +T*+T*+G*C*G*G*A*T*T*G*C*G*T*+C*+A*+C |
| 219 | A34217Hi | GAGATTGCGGATTGCGT | +G*+A*+G*A*T*T*G*C*G*G*A*T*T*G*+C*+G*+T |
| 220 | A34218Hi | AGATTGCGGATTGCGT | +A*+G*+A*T*T*G*C*G*G*A*T*T*G*+C*+G*+T |
| 221 | A34219Hi | CGTCAGTAATTTGGAGT | +C*+G*+T*C*A*G*T*A*A*T*T*T*G*G*+A*+G*+T |
| 222 | Neg1 | | +C*+G*+T*T*T*A*G*G*C*T*A*T*G*T*A*+C*+T*+T |

The oligonucleotides of the present invention hybridize for example with mRNA of human MTDH of SEQ ID NO.1 and/or introns of the pre-mRNA of human MTDH of SEQ ID NO.2. Such oligonucleotides are called MTDH antisense oligonucleotides. In some embodiments, the oligonucleotides hybridize within a hybridizing active area which is one or more region(s) on the MTDH mRNA, e.g., of SEQ ID NO.1 and/or the MTDH pre-mRNA, e.g., of SEQ ID NO.2, where hybridization with an oligonucleotide highly likely results in a potent knockdown of the MTDH expression. In the present invention surprisingly several hybridizing active regions were identified for example selected from hybridizing active regions for example selected from position 8500 to 8999, position 9000 to 9499, position 9500 to 9999, position 1000 to 10499, position 10500 to 10999, position 11000 to 11499, position 13000 to 13499, position 14000 to 14499, position 15500 to 15999, position 16500 to 16999, position 17500 to 17999, position 18000 to 18499, position 20500 to 20999, position 21000 to 21499, position 22500 to 22999, position 24000 to 24499, position 25000 to 25499, position 25500 to 25999, position 27000 to 27499, position 29000 to 29499, position 29500 to 29999, position 32000 to 32499, position 37000 to 37499, position 37500 to 37999, position 43500 to 43999, position 44500 to 44999, position 45500 to 45999, position 46500 to 46999, position 47000 to 47499, position 49000 to 49499, position 50500 to 50999, position 52000 to 52499, position 52500 to 52999, position 54000 to 54499, position 55500 to 55999, position 61500 to 61999, position 64000 to 64499, position 64500 to 64999, position 65000 to 65499, position 65500 to 65999, position 68000 to 68499, position 68500 to 68999, position 71500 to 71999, position 72000 to 72499, position 74500 to 74999, position 76000 to 76499, position 77000 to 77499, position 77500 to 77999, position 78000 to 78499, position 80500 to 80999, position 81000 to 81499, position 81500 to 81999, position 82000 to 82499, position 83500 to 83999, position 85000 to 85499, position 86000 to 86499, position 87500 to 87999, position 88500 to 88999, position 89000 to 89499, position 89500 to 89999, position 90000 to 90499, position 90500 to 90999, position 91000 to 91499, position 92000 to 92499, position 92500 to 92999, position 93500 to 93999, position 94500 to 94999 or a combination thereof (including the terminal figures of the ranges) of MTDH pre-mRNA for example of SEQ ID NO.2. Antisense oligonucleotides hybridizing with these regions are indicated in the following Table 2:

**Table 2 shows some hybridizing active regions and antisense oligonucleotides hybridizing in this region.**

| Region of SEQ ID NO.2 / ASO name | First position on SEQ ID NO.2 | SEQ ID NO. |
|---|---|---|
| **Region 8500-8999** | | |
| A34001H | 8844 | 3 |
| A34002H | 8850 | 4 |
| A34003H | 8850 | 5 |
| A340105H | 8843 | 107 |
| A340106H | 8844 | 108 |
| A340107H | 8851 | 109 |
| A340108H | 8851 | 110 |

| **Region 9000-9499** | | |
|---|---|---|
| A34004H | 9104 | 6 |
| A34005H | 9237 | 7 |
| A34006H | 9258 | 8 |
| A34007H | 9292 | 9 |
| A34008H | 9486 | 10 |
| A340109HM | 9238 | 111 |
| A340110HM | 9239 | 112 |
| A340111H | 9291 | 113 |

| **Region 9500-9999** | | |
|---|---|---|
| A34035Hi | 9564 | 37 |
| A34036Hi | 9649 | 38 |
| A34037Hi | 9732 | 39 |
| A34038Hi | 9837 | 40 |
| A34039Hi | 9938 | 41 |
| A34040Hi | 9999 | 42 |
| A34127Hi | 9651 | 129 |
| A34128Hi | 9652 | 130 |
| A34129Hi | 9731 | 131 |
| A34130Hi | 9833 | 132 |
| A34131Hi | 9841 | 133 |

| **Region 10000-10499** | | |
|---|---|---|
| A34041Hi | 10050 | 43 |
| A34042Hi | 10127 | 44 |
| A34043Hi | 10162 | 45 |
| A34044Hi | 10215 | 46 |
| A34045Hi | 10321 | 47 |
| A34132Hi | 10050 | 134 |
| A34133Hi | 10168 | 135 |
| A34134Hi | 10172 | 136 |
| A34135Hi | 10173 | 137 |
| A34136Hi | 10210 | 138 |
| A34138Hi | 10216 | 140 |
| A34139Hi | 10216 | 141 |
| A34140Hi | 10219 | 142 |
| A34141Hi | 10220 | 143 |
| A34142Hi | 10221 | 144 |
| A34143Hi | 10249 | 145 |
| A34144Hi | 10318 | 146 |
| A34145Hi | 10319 | 147 |

| **Region 10500-10999** | | |
|---|---|---|
| A34047Hi | 10538 | 49 |
| A34048Hi | 10576 | 50 |
| A34049Hi | 10635 | 51 |
| A34051Hi | 10902 | 53 |
| A34146Hi | 10573 | 148 |
| A34147Hi | 10575 | 149 |
| A34148Hi | 10637 | 150 |

| **Region 11000-11499** | | |
|---|---|---|
| A34052Hi | 11270 | 54 |
| A34053Hi | 11316 | 55 |
| A34054Hi | 11319 | 56 |
| A34055Hi | 11418 | 57 |
| A34150Hi | 11309 | 152 |
| A34151Hi | 11312 | 153 |
| A34152Hi | 11316 | 154 |
| A34153Hi | 11317 | 155 |
| A34154Hi | 11317 | 156 |
| A34155Hi | 11318 | 157 |
| A34156Hi | 11318 | 158 |
| A34157Hi | 11319 | 159 |
| A34158Hi | 11321 | 160 |
| A34159Hi | 11321 | 161 |
| A34160Hi | 11414 | 162 |
| A34161Hi | 11415 | 163 |
| A34162Hi | 11419 | 164 |

| **Region 13000-13499** | | |
|---|---|---|
| A34059Hi | 13208 | 61 |

| **Region 14000-14499** | | |
|---|---|---|
| A34164Hi | 14032 | 166 |

| **Region 15500-15999** | | |
|---|---|---|
| A34061Hi | 15672 | 63 |

| **Region 16500-16999** | | |
|---|---|---|
| A34064Hi | 16779 | 66 |
| A34165Hi | 16778 | 167 |
| A34166Hi | 16780 | 168 |
| A34167Hi | 16782 | 169 |

| **Region 17500-17999** | | |
|---|---|---|
| A34168Hi | 17990 | 170 |

| **Region 18000-18499** | | |
|---|---|---|
| A34065Hi | 18193 | 67 |

| **Region 20500-20999** | | |
|---|---|---|
| A34172Hi | 20810 | 174 |

| **Region 21000-21499** | | |
|---|---|---|
| A34069Hi | 21257 | 71 |

| **Region 22500-22999** | | |
|---|---|---|
| A34076Hi | 22958 | 78 |
| A34185Hi | 22954 | 187 |
| A34186Hi | 22951 | 188 |
| A34187Hi | 22952 | 189 |
| A34188Hi | 22953 | 190 |
| A34190Hi | 22956 | 192 |
| A34191Hi | 22961 | 193 |
| A34192Hi | 22964 | 194 |
| A34193Hi | 22965 | 195 |

| **Region 24000-24499** | | |
|---|---|---|
| A34078Hi | 24443 | 80 |

| **Region 25000-25499** | | |
|---|---|---|
| A34079Hi | 25369 | 81 |
| A34194Hi | 25362 | 196 |

| **Region 25500-25999** | | |
|---|---|---|
| A34009H | 25742 | 11 |
| A340112H | 25741 | 114 |

| **Region 27000-27499** | | |
|---|---|---|
| A34050Hi | 27086 | 52 |

| **Region 29000-29499** | | |
|---|---|---|
| A34058Hi | 29054 | 60 |
| A34163Hi | 29045 | 165 |

| **Region 29500-29999** | | |
|---|---|---|
| A34060Hi | 29798 | 62 |

| **Region 32000-32499** | | |
|---|---|---|
| A34062Hi | 32217 | 64 |
| A34063Hi | 32290 | 65 |

| **Region 37000-37499** | | |
|---|---|---|
| A34067Hi | 37441 | 69 |
| A34169Hi | 37438 | 171 |
| A34170Hi | 37439 | 172 |
| A34171Hi | 37440 | 173 |

| **Region 37500-37999** | | |
|---|---|---|
| A34068Hi | 37598 | 70 |
| A34070Hi | 37675 | 72 |
| A34071Hi | 37753 | 73 |
| A34173Hi | 37539 | 175 |
| A34174Hi | 37540 | 176 |
| A34175Hi | 37601 | 177 |
| A34176Hi | 37603 | 178 |
| A34177Hi | 37657 | 179 |
| A34178Hi | 37658 | 180 |
| A34179Hi | 37671 | 181 |
| A34180Hi | 37672 | 182 |
| A34181Hi | 37676 | 183 |
| A34182Hi | 37681 | 184 |
| A34183Hi | 37741 | 185 |

| **Region 43500-43999** | | |
|---|---|---|
| A34083Hi | 43635 | 85 |
| A34084Hi | 43723 | 86 |
| A34196Hi | 43631 | 198 |
| A34197Hi | 43633 | 199 |

| **Region 44500-44999** | | |
|---|---|---|
| A34085Hi | 44960 | 87 |

| **Region 45500-45999** | | |
|---|---|---|
| A34087Hi | 45815 | 89 |
| A34198Hi | 45817 | 200 |

| **Region 46500-46999** | | |
|---|---|---|
| A34089Hi | 46545 | 91 |
| A34199Hi | 46540 | 201 |
| A34200Hi | 46543 | 202 |

| **Region 47000-47499** | | |
|---|---|---|
| A34090Hi | 47441 | 92 |
| A34201Hi | 47439 | 203 |
| A34202Hi | 47443 | 204 |

| **Region 49000-49499** | | |
|---|---|---|
| A34091Hi | 49327 | 93 |
| A34203Hi | 49322 | 205 |
| A34206Hi | 49328 | 208 |
| A34207Hi | 49332 | 209 |

| **Region 50500-50999** | | |
|---|---|---|
| A34096Hi | 50651 | 98 |
| A34208Hi | 50650 | 210 |

| **Region 52000-52499** | | |
|---|---|---|
| A34010H | 52132 | 12 |
| A34113HM | 52206 | 115 |
| A3414HM | 52210 | 116 |
| A34115H | 52222 | 117 |
| A34011HM | 52212 | 13 |
| A34012H | 52221 | 14 |
| A34137Hi | 52428 | 139 |

| **Region 52500-52999** | | |
|---|---|---|
| A34046Hi | 52534 | 48 |
| A34149Hi | 52809 | 151 |

| **Region 54000-54499** | | |
|---|---|---|
| A34056Hi | 54009 | 58 |
| A34057Hi | 54285 | 59 |

| **Region 55500-55999** | | |
|---|---|---|
| A34013H | 55816 | 15 |

| **Region 61500-61999** | | |
|---|---|---|
| A34066Hi | 61845 | 68 |

| **Region 64000-64499** | | |
|---|---|---|
| A34116HM | 64490 | 118 |
| A34072Hi | 64330 | 74 |

| **Region 64500-64999** | | |
|---|---|---|
| A34073Hi | 64958 | 75 |
| A34184Hi | 64963 | 186 |

| **Region 65000-65499** | | |
|---|---|---|
| A34074Hi | 65234 | 76 |

| **Region 65500-65999** | | |
|---|---|---|
| A34075Hi | 65535 | 77 |
| A34077Hi | 65685 | 79 |
| A34189Hi | 65538 | 191 |

| **Region 68000-68499** | | |
|---|---|---|
| A34080Hi | 68180 | 82 |
| A34195Hi | 68481 | 197 |

| **Region 68500-68999** | | |
|---|---|---|
| A34081Hi | 68654 | 83 |
| A34082Hi | 68927 | 84 |

| **Region 71500-71999** | | |
|---|---|---|
| A34086Hi | 71718 | 88 |

| **Region 72000-72499** | | |
|---|---|---|
| A34088Hi | 72396 | 90 |

| **Region 74500-74999** | | |
|---|---|---|
| A34092Hi | 74688 | 94 |
| A34093Hi | 74876 | 95 |
| A34094Hi | 74933 | 96 |
| A34204Hi | 74687 | 206 |
| A34205Hi | 74877 | 207 |

| **Region 76000-76499** | | |
|---|---|---|
| A34095Hi | 76285 | 97 |

| **Region 77000-77499** | | |
|---|---|---|
| A34097Hi | 77266 | 99 |

| **Region 77500-77999** | | |
|---|---|---|
| A34209Hi | 77568 | 211 |

| **Region 78000-78499** | | |
|---|---|---|
| A34098Hi | 78105 | 100 |
| A34099Hi | 78457 | 101 |
| A34210Hi | 78109 | 212 |
| A34211Hi | 78110 | 213 |
| A34212Hi | 78458 | 214 |
| A34213Hi | 78458 | 215 |

| **Region 80500-80999** | | |
|---|---|---|
| A34100Hi | 80503 | 102 |

| **Region 81000-81499** | | |
|---|---|---|
| A34214Hi | 81204 | 216 |

| **Region 81500-81999** | | |
|---|---|---|
| A34101Hi | 81622 | 103 |

| **Region 82000-82499** | | |
|---|---|---|
| A34102Hi | 82216 | 104 |

| **Region 83500-83999** | | |
|---|---|---|
| A340117H | 83822 | 119 |
| A340118H | 83823 | 120 |
| A340119H | 83832 | 121 |
| A34014H | 83814 | 16 |
| A34015H | 83817 | 17 |
| A34016H | 83821 | 18 |
| A34017H | 83825 | 19 |

| **Region 85000-85499** | | |
|---|---|---|
| A34103Hi | 85364 | 105 |
| A34215Hi | 85363 | 217 |
| A34216Hi | 85364 | 218 |
| A34217Hi | 85367 | 219 |
| A34218Hi | 85367 | 220 |

| **Region 86000-86499** | | |
|---|---|---|
| A34219Hi | 86343 | 221 |

| **Region 87500-87999** | | |
|---|---|---|
| A34018H | 87626 | 20 |
| A34019H | 87626 | 21 |

| **Region 88500-88999** | | |
|---|---|---|
| A34104Hi | 88632 | 106 |

| **Region 89000-89499** | | |
|---|---|---|
| A34120H | 89314 | 122 |
| A34020H | 89313 | 22 |
| A34021H | 89315 | 23 |

| **Region 89500-89999** | | |
|---|---|---|
| A34022H | 89562 | 24 |

| **Region 90000-90499** | | |
|---|---|---|
| A34121H | 90312 | 123 |
| A34023H | 90313 | 25 |
| A34024H | 90320 | 26 |

| **Region 90500-90999** | | |
|---|---|---|
| A34122HM | 90556 | 124 |
| A34025HM | 90554 | 27 |
| A34026H | 90661 | 28 |
| A34027H | 90746 | 29 |

| **Region 91000-91499** | | |
|---|---|---|
| A34028H | 91356 | 30 |

| **Region 92000-92499** | | |
|---|---|---|
| A34029H | 92491 | 31 |
| A34030H | 92498 | 32 |

| **Region 92500-92999** | | |
|---|---|---|
| A34123H | 92503 | 125 |
| A34031H | 92502 | 33 |

| **Region 93500-93999** | | |
|---|---|---|
| A34124H | 93935 | 126 |
| A34125H | 93942 | 127 |
| A34032H | 93933 | 34 |
| A34033H | 93940 | 35 |

| **Region 94500-94999** | | |
|---|---|---|
| A34126H | 94647 | 128 |
| A34034H | 94648 | 36 |

The following Table 3 presents embodiments of oligonucleotides comprising modified nucleotides for example LNA which are indicated by (+) and phosphorothioate (PTO) indicated by (*). The oligonucleotides consisting of or comprising the sequences of Table 3 may comprise any other modified nucleotide and/or any other combination of modified and unmodified nucleotides. Oligonucleotides of Table 3 hybridize with exonic regions of the mRNA of mouse MTDH (SEQ ID NO.223; NM_001357926.1) or with intronic regions of the pre-mRNA of mouse MTDH (SEQ ID NO.224; chr15:34076600-34149700), indicated by "i" in the following Table 3:

**Table 3: List of antisense oligonucleotides hybridizing with mouse MTDH mRNA for example of SEQ ID NO.223 and / or intronic regions of the mouse MTDH pre-mRNA (Mi) for example of SEQ ID NO.224. The antisense oligonucleotides were designed according to in house criteria and negl (described in WO2014154843 A1) was used as a control oligonucleotide in all experiments.**

| Seq ID | Name | Antisense Sequence 5'-3' | Antisense Sequence 5'-3' with PTO (*) and LNA (+) |
|---|---|---|---|
| 225 | A34001M | CGAGCGCATCGCGGCCA | +C*+G*+A*G*C*G*C*A*T*C*G*C*G*G*+C*+C*+A |
| 226 | A34002M | CCGCTGGAACAGTCGTG | +C*+C*G*C*T*G*G*A*A*C*A*G*T*C*+G*+T*+G |
| 227 | A34003M | CCGTCAGAGAGACTCGC | +C*+C*+G*T*C*A*G*A*G*A*G*A*C*T*+C*+G*+C |
| 228 | A34004M | CCGACCGAGAGCAACTC | +C*+C*+G*A*C*C*G*A*G*A*G*C*A*A*+C*+T*+C |
| 229 | A34005M | AAACCTAGGCCGACCGA | +A*+A*+A*C*C*T*A*G*G*C*C*G*A*C*+C*+G*+A |
| 230 | A34006M | AAACCTAGGCCGACCG | +A*+A*+A*C*C*T*A*G*G*C*C*G*A*+C*+C*+G |
| 231 | A34007M | AAAACCTAGGCCGACCG | +A*+A*+A*A*C*C*T*A*G*G*C*C*G*A*+C*+C*+G |
| 232 | A34008M | CCGTGCGCAGAAAACCT | +C*+C*+G*T*G*C*G*C*A*G*A*A*A*A*+C*+C*+T |
| 233 | A34009M | AACTCCGTGCGCAGAAA | +A*+A*+C*T*C*C*G*T*G*C*G*C*A*G*+A*+A*+A |
| 234 | A34010M | TACCGCTTCGGCTCTAG | +T*+A*+C*C*G*C*T*T*C*G*G*C*T*C*+T*+A*+G |
| 235 | A34011M | TACCGCTTCGGCTCTA | +T*+A*+C*C*G*C*T*T*C*G*G*C*T*+C*+T*+A |
| 236 | A34012M | GTACCGCTTCGGCTCTA | +G*+T*+A*C*C*G*C*T*T*C*G*G*C*T*+C*+T*+A |
| 237 | A34013M | AGCCGTAACCTAGAAGG | +A*+G*+C*C*G*T*A*A*C*C*T*A*G*A*+A*+G*+G |
| 238 | A34014M | CTCCTTCGCTTCTTGCG | +C*+T*+C*C*T*T*C*G*C*T*T*C*T*T*G*+C*+G |
| 239 | A34015M | CAACAGTCCGTCCATTT | +C*+A*+A*C*A*G*T*C*C*G*T*C*C*A*+T*+T*+T |
| 240 | A34016M | TCAACAGTCCGTCCATT | +T*+C*+A*A*C*A*G*T*C*C*G*T*C*C*+A*+T*+T |
| 241 | A34017M | GTACTTCAACAGTCCGT | +G*+T*+A*C*T*T*C*A*A*C*A*G*T*C*+C*+G*+T |
| 242 | A34018M | GTACTTCAACAGTCCG | +G*+T*+A*C*T*T*C*A*A*C*A*G*T*+C*+C*+G |
| 243 | A34019M | GGTACTTCAACAGTCCG | +G*+G*+T*A*C*T*T*C*A*A*C*A*G*T*+C*+C*+G |
| 244 | A34020HM | ATCATGGCGTGAACTGT | +A*+T*C*A*T*G*G*C*G*T*G*A*A*C*+T*+G*+T |
| 245 | A34021M | CGCTGTTGTCGTTTCTC | +C*+G*+C*T*G*T*T*G*T*C*G*T*T*T*+C*+T*+C |
| 246 | A34022M | GTTTACGCTGTTGTCG | +G*+T*+T*T*A*C*G*C*T*G*T*T*G*+T*+C*+G |
| 247 | A34023M | TCACGTTTACGCTGTTG | +T*+C*+A*C*G*T*T*T*A*C*G*C*T*G*+T*+T*+G |
| 248 | A34024M | TATCACGTTTACGCTGT | +T*+A*+T*C*A*C*G*T*T*T*A*C*G*C*+T*+G*+T |
| 249 | A34025HM | TGTAAGTTGCTCGGTGG | +T*+G*+T*A*A*G*T*T*G*C*T*C*G*G*+T*+G*+G |
| 250 | A34026M | AGTTGTAAGTTGCTCGG | +A*+G*+T*T*G*T*A*A*G*T*T*G*C*T*+C*+G*+G |
| 251 | A34027M | GAAAATATTGAGCGATC | +G*+A*+A*A*A*T*A*T*T*G*A*G*C*G*+A*+T*+C |
| 252 | A34028HM | GTTGATTACGGCTAA | +G*+T*+T*G*A*T*T*A*C*G*G*C*+T*+A*+A |
| 253 | A34029HM | GGTTGATTACGGCTAA | +G*+G*+T*T*G*A*T*T*A*C*G*G*C*+T*+A*+A |
| 254 | A34030M | AGGTTGATTACGGCTAA | +A*+G*+G*T*T*G*A*T*T*A*C*G*G*C*+T*+A*+A |
| 255 | A34031M | ATCATCGATATAAGGTT | +A*+T*+C*A*T*C*G*A*T*A*T*A*A*G*+G*+T*+T |
| 256 | A34032M | GTAATAGATGGCTCGGC | +G*+T*+A*A*T*A*G*A*T*G*G*C*T*C*+G*+G*+C |
| 257 | A34033M | TAACATCGGTGGCACTT | +T*+A*+A*C*A*T*C*G*G*T*G*G*C*A*+C*+T*+T |
| 258 | A34034M | GATCATTCTCGTTCAAT | +G*+A*+T*C*A*T*T*C*T*C*G*T*T*C*+A*+A*+T |
| 259 | A34035M | AAGGTCGTGCATAAGAT | +A*+A*+G*G*T*C*G*T*G*C*A*T*A*A*+G*+A*+T |
| 260 | A34036M | AAGGTCGTGCATAAGA | +A*+A*+G*G*T*C*G*T*G*C*A*T*A*+A*+G*+A |
| 261 | A34037M | TCTAAGGTCGTGCATAA | +T*+C*+T*A*A*G*G*T*C*G*T*G*C*A*+T*+A*+A |
| 262 | A34038M | TCTAAGGTCGTGCATA | +T*+C*+T*A*A*G*G*T*C*G*T*G*C*+A*+T*+A |
| 263 | A34039M | GGTCTAAGGTCGTGCAT | +G*+G*+T*C*T*A*A*G*G*T*C*G*T*G*+C*+A*+T |
| 264 | A34040M | TCCGTCATATGCTCAAA | +T*+C*+C*G*T*C*A*T*A*T*G*C*T*C*+A*+A*+A |
| 265 | A34041M | CTCCGTCATATGCTCAA | +C*+T*+C*C*G*T*C*A*T*A*T*G*C*T*+C*+A*+A |
| 266 | A34042M | TAACCGGAGGTCTCCAC | +T*+A*+A*C*C*G*G*A*G*G*T*C*T*C*C*+A*+C |
| 267 | A34043M | AGATCATACGTCATTCA | +A*+G*+A*T*C*A*T*A*C*G*T*C*A*T*+T*+C*+A |
| 268 | A34044M | ACAGATCATACGTCATT | +A*+C*+A*G*A*T*C*A*T*A*C*G*T*C*+A*+T*+T |
| 269 | A34045M | TGTACATGGCCGAGCAT | +T*+G*+T*A*C*A*T*G*G*C*C*G*A*G*+C*+A*+T |
| 270 | A34046HM | CGTTTGGTAAAGGCTAT | +C*+G*+T*T*T*G*G*T*A*A*A*G*G*C*+T*+A*+T |
| 271 | A34047M | CCTTCTACCGGCGTGG | +C*+C*+T*T*C*T*A*C*C*G*G*C*G*+T*+G*+G |
| 272 | A34048M | CCTTCTACCGGCGTG | +C*+C*+T*T*C*T*A*C*C*G*G*C*+G*+T*+G |
| 273 | A34049M | CCTCCTTCTACCGGCG | +C*+C*+T*C*C*T*T*C*T*A*C*C*G*+G*+C*+G |
| 274 | A34050Mi | GGACATTGGCTCAATC | +G*+G*+A*C*A*T*T*G*G*C*T*C*A*+A*+T*+C |
| 275 | A34051Mi | ACGCATCTGAGTACTTG | +A*+C*+G*C*A*T*C*T*G*A*G*T*A*C*+T*+T*+G |
| 276 | A34052Mi | TTGCTTACGCATCTGAG | +T*+T*+G*C*T*T*A*C*G*C*A*T*C*T*+G*+A*+G |
| 277 | A34053Mi | ACGAGGTTAAGAAGGTG | +A*+C*+G*A*G*G*T*T*A*A*G*A*A*G*+G*+T*+G |
| 278 | A34054Mi | TACCAAACTCCGAGTGT | +T*+A*+C*C*A*A*A*C*T*C*C*G*A*G*+T*+G*+T |
| 279 | A34055Mi | TGATTGATTCCGAGAT | +T*+G*+A*T*T*G*A*T*T*C*C*G*A*+G*+A*+T |
| 280 | A34056Mi | ACGTAGCTTACATAAGT | +A*+C*+G*T*A*G*C*T*T*A*C*A*T*A*+A*+G*+T |
| 281 | A34057Mi | AACGATATAGCTAGCAC | +A*+A*+C*G*A*T*A*T*A*G*C*T*A*G*+C*+A*+C |
| 282 | A34058Mi | GTAAGTAAGAATACGAT | +G*+T*+A*A*G*T*A*A*G*A*A*T*A*C*+G*+A*+T |
| 283 | A34059Mi | ACGATGGTAGGAATTTA | +A*+C*+G*A*T*G*G*T*A*G*G*A*A*T*+T*+T*+A |
| 284 | A34060Mi | CACGTTGGTCAATAGTA | +C*+A*+C*G*T*T*G*G*T*C*A*A*T*A*G*+T*+A |
| 285 | A34061Mi | GACGAGGAGTTAACAAA | +G*+A*+C*G*A*G*G*A*G*T*T*A*A*C*+A*+A*+A |
| 286 | A34062Mi | TAGTATATTAGCCACTC | +T*+A*+G*T*A*T*A*T*T*A*G*C*C*A*+C*+T*+C |
| 287 | A34063Mi | AGACGGATTGCTGATAT | +A*+G*+A*C*G*G*A*T*T*G*C*T*G*A*+T*+A*+T |
| 288 | A34064Mi | TGTAGACGGATTGCTGA | +T*+G*+T*A*G*A*C*G*G*A*T*T*G*C*+T*+G*+A |
| 289 | A34065Mi | GTTATGTAGACGGATT | +G*+T*+T*A*T*G*T*A*G*A*C*G*G*+A*+T*+T |
| 290 | A34066Mi | CATGCGCATTAAAGAGT | +C*+A*+T*G*C*G*C*A*T*T*A*A*A*G*+A*+G*+T |
| 291 | A34067Mi | AATTGCTCTACGGCTAC | +A*+A*+T*T*G*C*T*C*T*A*C*G*G*C*+T*+A*+C |
| 292 | A34068Mi | ACCAATTGCTCTACGGC | +A*+C*+C*A*A*T*T*G*C*T*C*T*A*C*+G*+G*+C |
| 293 | A34069Mi | TTTCTTAGGAACCGGAT | +T*+T*+T*C*T*T*A*G*G*A*A*C*C*G*+G*+A*+T |
| 294 | A34070Mi | CATCGCTAGACGCCTCG | +C*+A*+T*C*G*C*T*A*G*A*C*G*C*C*+T*+C*+G |
| 295 | A34071Mi | AACAGCCTCATCGCTAG | +A*+A*+C*A*G*C*C*T*C*A*T*C*G*C*+T*+A*+G |
| 296 | A34072Mi | GACTTGTCGTCTGCAAA | +G*+A*C*T*T*G*T*C*G*T*C*T*G*C*+A*+A*+A |
| 297 | A34073Mi | GTAGTATACAGTTGGTG | +G*+T*+A*G*T*A*T*A*C*A*G*T*T*G*+G*+T*+G |
| 298 | A34074Mi | AACACCGCCTTAGTCAT | +A*+A*+C*A*C*C*G*C*C*T*T*A*G*T*+C*+A*+T |
| 299 | A34075Mi | ACCTTATAAGTTCGTAA | +A*+C*+C*T*T*A*T*A*A*G*T*T*C*G*+T*+A*+A |
| 300 | A34076Mi | GGCGAACCTTATAAGT | +G*+G*+C*G*A*A*C*C*T*T*A*T*A*+A*+G*+T |
| 301 | A34077Mi | GCTTGTGCGGTGCTCTC | +G*+C*+T*T*G*T*G*C*G*G*T*G*C*T*+C*+T*+C |
| 302 | A34078Mi | CCTCGATGTTAGATGTT | +C*+C*+T*C*G*A*T*G*T*T*A*G*A*T*+G*+T*+T |
| 303 | A34079Mi | ACTACTCGGATTTCACT | +A*+C*+T*A*C*T*C*G*G*A*T*T*T*C*+A*+C*+T |
| 304 | A34080Mi | TTCGGACTATTAAACCA | +T*+T*+C*G*G*A*C*T*A*T*T*A*A*A*+C*+C*+A |
| 305 | A34081Mi | TTCGCTCACAGCAGACC | +T*+T*C*G*C*T*C*A*C*A*G*C*A*G*A*+C*+C |
| 306 | A34082Mi | AGTATTAATATAGCGGT | +A*+G*+T*A*T*T*A*A*T*A*T*A*G*C*+G*+G*+T |
| 307 | A34083Mi | GCCGATGAATATTCAGT | +G*+C*+C*G*A*T*G*A*A*T*A*T*T*C*+A*+G*+T |
| 308 | A34084Mi | ATGTATTATGAGCCGAT | +A*+T*+G*T*A*T*T*A*T*G*A*G*C*C*+G*+A*+T |
| 309 | A34085Mi | GTGCAACAAGTGATTAC | +G*+T*+G*C*A*A*C*A*A*G*T*G*A*T*+T*+A*+C |
| 310 | A34086Mi | GTTAAGTTGGTCCAAGA | +G*+T*+T*A*A*G*T*T*G*G*T*C*C*A*+A*+G*+A |
| 311 | A34087Mi | TCGACTGATCTGTTCCA | +T*+C*+G*A*C*T*G*A*T*C*T*G*T*T*+C*+C*+A |
| 312 | A34088Mi | CCGTTCGACTGATCTGT | +C*+C*+G*T*T*C*G*A*C*T*G*A*T*C*+T*+G*+T |
| 313 | A34089Mi | TACACCGTTCGACTGA | +T*+A*+C*A*C*C*G*T*T*C*G*A*C*+T*+G*+A |
| 314 | A34090Mi | TTTCTACACCGTTCGA | +T*+T*+T*C*T*A*C*A*C*C*G*T*T*+C*+G*+A |
| 315 | A34091Mi | TCGGCAAACCATTCACT | +T*+C*+G*G*C*A*A*A*C*C*A*T*T*C*+A*+C*+T |
| 316 | A34092Mi | GCTAAGTTAACGCTTCC | +G*+C*+T*A*A*G*T*T*A*A*C*G*C*T*+T*+C*+C |
| 317 | A34093Mi | TGGTCTATGCTAGAGGT | +T*+G*+G*T*C*T*A*T*G*C*T*A*G*A*+G*+G*+T |
| 318 | A34094Mi | CTCGTGGTATGACTAAG | +C*+T*+C*G*T*G*G*T*A*T*G*A*C*T*+A*+A*+G |
| 319 | A34095Mi | AAGCATCCGACCTTTGG | +A*+A*+G*C*A*T*C*C*G*A*C*C*T*T*+T*+G*+G |
| 320 | A34096Mi | TTCGGTAGCTAGTCTGC | +T*+T*+C*G*G*T*A*G*C*T*A*G*T*C*+T*+G*+C |
| 321 | A34097Mi | GGAAGTGGTCCACGATA | +G*+G*+A*A*G*T*G*G*T*C*C*A*C*G*+A*+T*+A |
| 322 | A34098Mi | AGGCGACGGAGGAGTTA | +A*+G*+G*C*G*A*C*G*G*A*G*G*A*G*+T*+T*+A |
| 323 | A34099Mi | TATACTAACGCAGATCC | +T*+A*+T*A*C*T*A*A*C*G*C*A*G*A*+T*+C*+C |
| 324 | A34100Mi | ATATACTAACGCAGATC | +A*+T*+A*T*A*C*T*A*A*C*G*C*A*G*+A*+T*+C |
| 325 | A34101Mi | GTAGCAGCGTGTACATT | +G*+T*+A*G*C*A*G*C*G*T*G*T*A*C*+A*+T*+T |
| 326 | A34102Mi | AGTGGTAGCAGCGTGTA | +A*+G*+T*G*G*T*A*G*C*A*G*C*G*T*G*+T*+A |
| 327 | A34103Mi | CTCTACTATCGCACCAG | +C*+T*+C*T*A*C*T*A*T*C*G*C*A*C*+C*+A*+G |
| 328 | A34104Mi | GCTCTACTATCGCACC | +G*+C*+T*C*T*A*C*T*A*T*C*G*C*+A*+C*+C |
| 222 | Neg 1 | | +C*+G*+T*T*T*A*G*G*C*T*A*T*G*T*A*+C*+T*+T |

The oligonucleotide of the present invention inhibits for example at least about 50%, 55%, 60%, 65%, 70%, 75%, 80%, 85%, 90%, 92%, 94%, 95%, 96%, 97%, 98%, 99% or 100% of MTDH expression such as the, e.g., human, rat or murine, MTDH expression. The oligonucleotides of the present invention are for example active and inhibit expression for example in a cell, tissue, organ, or a subject. The oligonucleotide of the present invention inhibits the expression of MTDH at a nanomolar or micromolar concentration for example in a concentration of 0.1, 1, 2, 3, 4, 5, 6, 7, 8, 9, 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900 or 950 nM, or 1, 10 or 100 µM.

The oligonucleotide of the present invention is for example used in a concentration of 1, 3, 5, 9, 10, 15, 27, 30, 40, 50, 75, 82, 100, 250, 300, 500, or 740 nM, or 1, 2.2, 3, 5, 6.6 or 10 µM.

In some embodiments the present invention refers to a pharmaceutical composition comprising an oligonucleotide of the present invention and a pharmaceutically acceptable carrier, excipient and/or dilutant. The pharmaceutical composition further comprises for example a chemotherapeutic, another disease specific active agent such as insulin, angiotensin-converting enzyme inhibitor, angiotensin receptor blocker, another oligonucleotide, an antibody, a peptide-based therapeutic, a protein-based therapeutic, a HERA fusion protein, a ligand trap, a Fab fragment, a nanobody, a BiTe and/or a small molecule which is for example effective in tumor treatment, or treatment of a kidney disease such as diabetic nephropathy.

In some embodiments, the oligonucleotide or the pharmaceutical composition of the present invention is for use in a method of preventing and/or treating a disorder. In some embodiments, the use of the oligonucleotide or the pharmaceutical composition of the present invention in a method of preventing and/or treating a disorder is combined with radiotherapy. The radiotherapy may be further combined with a chemotherapy (e.g., platinum, gemcitabine). The disorder is for example characterized by an MTDH imbalance, i.e., the MTDH level is increased in comparison to the level in a normal, healthy cell, tissue, organ or subject or MTDH expression for example is involved in the induction of the disease and/or mediates resistance to another therapy. The MTDH level is for example increased by an increased MTDH expression and activity, respectively. The MTDH level can be measured by any standard method such as immunohistochemistry, western blot, quantitative real time PCR or QuantiGene assay known to a person skilled in the art.

An oligonucleotide or a pharmaceutical composition of the present invention is for example administered locally or systemically for example orally, sublingually, nasally, inhaled, subcutaneously, intravenously, intraperitoneally, intramuscularly, intratumoral, intrathecal, transdermal, and/or rectal. Alternatively or in combination *ex vivo* treated immune cells are administered. The oligonucleotide is administered alone or in combination with another antisense oligonucleotide of the present invention and optionally in combination with another compound such as another oligonucleotide, an antibody, a carbohydrate-modified antibody, a peptide-based therapeutic, a protein-based therapeutic, a HERA fusion protein, a ligand trap, a Fab fragment, a nanobody, a BiTe, a small molecule and/or a chemotherapeutic (e.g., platinum, gemcitabine) and/or another disease specific agent such as insulin, angiotension-converting enzyme inhibitor, and/or angiotensin receptor blocker. In some embodiments, the other oligonucleotide (i.e., not being part of the present invention), the antibody, a peptide-based therapeutic, a protein-based therapeutic, a HERA fusion protein, a ligand trap, a Fab fragment, a nanobody, a BiTe, and/or the small molecule are effective in preventing and/or treating diabetes kidney disease such as diabetic nephropathy, artheriosclerosis, a nephrological disorder and/or cancer. An oligonucleotide or a pharmaceutical composition of the present invention is for example used in a method of preventing and/or treating a solid tumor or a hematologic tumor. Examples of cancers preventable and/or treatable by use of the oligonucleotide or pharmaceutical composition of the present invention are breast cancer, lung cancer, malignant melanoma, lymphoma, skin cancer, bone cancer, prostate cancer, liver cancer, brain cancer, cancer of the larynx, gall bladder, pancreas, testicular, rectum, parathyroid, thyroid, adrenal, neural tissue, head and neck, colon, stomach, bronchi, kidneys, basal cell carcinoma, squamous cell carcinoma, metastatic skin carcinoma, osteo sarcoma, Ewing's sarcoma, reticulum cell sarcoma, liposarcoma, myeloma, giant cell tumor, small-cell lung tumor, islet cell tumor, primary brain tumor, meningioma, acute and chronic lymphocytic and granulocytic tumors, acute and chronic myeloid leukemia, hairy-cell tumor, adenoma, hyperplasia, medullary carcinoma, intestinal ganglioneuromas, Wilm's tumor, seminoma, ovarian tumor, leiomyomater tumor, cervical dysplasia, retinoblastoma, soft tissue sarcoma, malignant carcinoid, topical skin lesion, rhabdomyosarcoma, Kaposi's sarcoma, osteogenic sarcoma, malignant hypercalcemia, renal cell tumor, polycythermia vera, adenocarcinoma, anaplastic astrocytoma, glioblastoma multiforma, leukemia, or epidermoid carcinoma.

Another example of a disease preventable and/or treatable by use of the oligonucleotide or pharmaceutical composition of the present invention other than cancer is for example diabetic, nephropathy.

In some embodiments two or more oligonucleotides of the present invention are administered together, at the same time point for example in a pharmaceutical composition or separately, or on staggered intervals. In other embodiments, one or more oligonucleotides of the present invention are administered together with another compound such as another oligonucleotide (i.e., not being part of the present invention), an antibody, a peptide-based therapeutic, a protein-based therapeutic, a HERA fusion protein, a ligand trap, a Fab fragment, a nanobody, a BiTe, a small molecule and/or a chemotherapeutic, at the same time point for example in a pharmaceutical composition or separately, or on staggered intervals. In some embodiments of these combinations, the antisense oligonucleotide inhibits the expression and activity, respectively, of MTDH and the other oligonucleotide (i.e., not being part of the present invention), the antibody, a peptide-based therapeutic, a protein-based therapeutic, a HERA fusion protein, a ligand trap, a Fab fragment, a nanobody, a BiTe and/or small molecule inhibits (antagonist) MTDH or inhibits or stimulates (agonist) an immune suppressive factor and/or an immune stimulatory factor or inhibits another target that is involved in cancer progression and/or metastasis. The immune suppressive factor is for example selected from the group consisting of IDOl, IDO2, CTLA-4, PD-1, PD-L1, LAG-3, VISTA, A2AR, CD39, CD73, STAT3, TDO2, TIM-3, TIGIT, TGF-beta, BTLA, MICA, NKG2A, KIR, CD160, MTDH, Xbp1 and a combination thereof. The immune stimulatory factor is for example selected from the group consisting of 4-1BB, Ox40, KIR, GITR, CD27, 2B4 and a combination thereof. The factor involved in cancer progression and/or metastasis is for example selected from the group consisting of SND1, HER-2, BRAF, VEGF, EGFR1, EGFR2, BCR/ABL, ABL, MET, ALK, JAK2, BTK, miR-223, CCL18, CCL20, Lcn2, CCL5/CCR9, DDR2, PHD2, IL6, SDF-1/CXCL12 and a combination thereof.

The immune suppressive factor is a factor whose expression and/or activity is for example increased in a cell, tissue, organ or subject. The immune stimulatory factor is a factor whose level is increased or decreased in a cell, tissue, organ or subject depending on the cell, tissue, organ or subject and its individual conditions. The factor involved in cancer progression and/or metastasis is a factor whose level is increased or decreased in a cell, tissue, organ or subject depending on the cell, tissue, organ or subject and its individual conditions in comparison to a healthy subject or is for example involved in the induction of the disease and/or mediates resistance to another therapy.

An antibody in combination with the oligonucleotide or the pharmaceutical composition of the present invention is for example an anti-PD-1 antibody, an anti-PD-Ll antibody, or a bispecific antibody. A small molecule in combination with the oligonucleotide or the pharmaceutical composition of the present invention are for example Sunitinib, Alecitinib, Afatinib, Ibrutinib, Imatinib, Lenvatinib, or Epacadostat. A chemotherapy in combination with the oligonucleotide or the pharmaceutical composition of the present invention is for example platinum or gemcitabine.

A subject of the present invention is for example a mammalian such as a dog, cat, horse, cow, pig etc., a bird or a fish.

### Examples

The following examples illustrate different embodiments of the present invention, but the invention is not limited to these examples. The following experiments are performed on cells endogenously expressing MTDH, i.e., the cells do not represent an artificial system comprising transfected reporter constructs. Such artificial systems generally show a higher degree of inhibition and lower IC₅₀ values than endogenous systems which are closer to therapeutically relevant *in vivo* systems. Further, in the following experiments no transfecting agent is used, i.e., gymnotic delivery is performed. Transfecting agents are known to increase the activity of an oligonucleotide which influences the IC₅₀ value (see for example Zhang et al., Gene Therapy, 2011, 18, 326-333; Stanton et al., Nucleic Acid Therapeutics, Vol. 22, No. 5, 2012). As artificial systems using a transfecting agent are hardly or impossible to translate into therapeutic approaches and no transfection formulation has been approved so far for oligonucleotides, the following experiments are performed without any transfecting agent.

### Example 1: Efficacy screen of MTDH antisense oligonucleotides in human cancer cell lines

In order to investigate the knockdown efficacy of the *in silico* designed MTDH antisense oligonucleotides, efficacy screening experiments were performed in two cell lines, namely EFO-21 and SKOV-3. Therefore, cells were treated with the respective antisense oligonucleotide at a concentration of 5 µM for three days without the use of a transfection reagent. Cells were lysed after three days treatment period, MTDH and HPRT1 mRNA expression was analyzed using the QuantiGene Singleplex assay (ThermoFisher) and the MTDH expression values were normalized to HPRT1 values. As shown in Figure 1 A) and Table 4, treatment of EFO-21 cells with 35 of 104 (34 %) antisense oligonucleotides resulted in a knockdown efficacy of > 80 % (represented by a residual MTDH mRNA expression of < 0.2 compared to mock-treated cells). Treatment with the control oligonucleotide negl had no relevant impact on MTDH mRNA expression. As shown in Figure 1 B) and Table 5, treatment of SKOV-3 cells with nine of 104 (9 %) antisense oligonucleotides resulted in a knockdown efficacy of > 70 % (represented by a residual MTDH mRNA expression of < 0.3 compared to mock-treated cells). Treatment with the control oligo negl had no relevant impact on MTDH mRNA expression. Tables 4 and 5 are shown in the following: c

**Table 4: List of the mean MTDH mRNA expression values in antisense oligonucleotide-treated EFO-21 cells compared to mock-treated cells. Expression values are normalized to HPRT1.**

| **ASO** | **Residual MTDH mRNA expression (compared to mock-treated cells)** |
|---|---|
| A34011HM | 0.04 |
| A34062Hi | 0.05 |
| A34082Hi | 0.05 |
| A34084Hi | 0.05 |
| A34048Hi | 0.05 |
| A34053Hi | 0.08 |
| A34061Hi | 0.08 |
| A34088Hi | 0.09 |
| A34027H | 0.09 |
| A34077Hi | 0.10 |
| A34065Hi | 0.11 |
| A34012H | 0.11 |
| A34026H | 0.11 |
| A34079Hi | 0.12 |
| A34094Hi | 0.12 |
| A34044Hi | 0.13 |
| A34052Hi | 0.13 |
| A34060Hi | 0.13 |
| A34095Hi | 0.14 |
| A34074Hi | 0.14 |
| A34018H | 0.14 |
| A34019H | 0.14 |
| A34047Hi | 0.15 |
| A34086Hi | 0.15 |
| A34068Hi | 0.15 |
| A34073Hi | 0.16 |
| A34071Hi | 0.16 |
| A34041Hi | 0.17 |
| A34025HM | 0.17 |
| A34076Hi | 0.17 |
| A34022H | 0.19 |
| A34050Hi | 0.19 |
| A34072Hi | 0.19 |
| A34066Hi | 0.20 |
| A34064Hi | 0.20 |
| A34038Hi | 0.22 |
| A34054Hi | 0.22 |
| A34083Hi | 0.22 |
| A34100Hi | 0.23 |
| A34030H | 0.23 |
| A34021H | 0.23 |
| A34078Hi | 0.23 |
| A34045Hi | 0.23 |
| A34039Hi | 0.24 |
| A34058Hi | 0.24 |
| A34029H | 0.25 |
| A34081Hi | 0.25 |
| A34067Hi | 0.27 |
| A34043Hi | 0.28 |
| A34091Hi | 0.28 |
| A34010H | 0.29 |
| A34016H | 0.29 |
| A34023H | 0.30 |
| A34031H | 0.31 |
| A34096Hi | 0.31 |
| A34098Hi | 0.32 |
| A34024H | 0.32 |
| A34090Hi | 0.33 |
| A34092Hi | 0.34 |
| A34080Hi | 0.34 |
| A34008H | 0.34 |
| A34032H | 0.36 |
| A34046Hi | 0.36 |
| A34034H | 0.36 |
| A34075Hi | 0.37 |
| A34049Hi | 0.37 |
| A34017H | 0.37 |
| A34087Hi | 0.38 |
| A34035Hi | 0.39 |
| A34057Hi | 0.40 |
| A34104Hi | 0.42 |
| A34056Hi | 0.43 |
| A34070Hi | 0.46 |
| A34059Hi | 0.50 |
| A34040Hi | 0.51 |
| A34013H | 0.51 |
| A34089Hi | 0.52 |
| A34037Hi | 0.53 |
| A34028H | 0.54 |
| A34055Hi | 0.54 |
| A34042Hi | 0.54 |
| A34009H | 0.55 |
| A34036Hi | 0.58 |
| A34007H | 0.60 |
| A34003H | 0.63 |
| A34069Hi | 0.63 |
| A34001H | 0.63 |
| A34033H | 0.64 |
| A34014H | 0.64 |
| A34063Hi | 0.67 |
| A34093Hi | 0.71 |
| A34051Hi | 0.72 |
| A34015H | 0.74 |
| A34020H | 0.74 |
| A34006H | 0.76 |
| A34103Hi | 0.77 |
| A34097Hi | 0.77 |
| A34102Hi | 0.82 |
| A34101Hi | 0.84 |
| A34002H | 0.86 |
| A34004H | 0.93 |
| A34005H | 1.10 |
| A34085Hi | 1.18 |
| Control oligo | 1.19 |
| A34099Hi | 1.20 |

**Table 5: List of the mean MTDH mRNA expression values in antisense oligonucleotide-treated SKOV-3 cells compared to mock-treated cells. Expression values are normalized to HPRT1.**

| **ASO** | **Residual MTDH mRNA expression (compared to mock-treated cells)** |
|---|---|
| A34011HM | 0.10 |
| A34026H | 0.18 |
| A34084Hi | 0.19 |
| A34025HM | 0.22 |
| A34082Hi | 0.25 |
| A34062Hi | 0.26 |
| A34077Hi | 0.28 |
| A34019H | 0.28 |
| A34012H | 0.30 |
| A34060Hi | 0.31 |
| A34052Hi | 0.31 |
| A34080Hi | 0.32 |
| A34061Hi | 0.32 |
| A34047Hi | 0.33 |
| A34018H | 0.33 |
| A34073Hi | 0.33 |
| A34058Hi | 0.33 |
| A34083Hi | 0.33 |
| A34044Hi | 0.34 |
| A34078Hi | 0.35 |
| A34100Hi | 0.35 |
| A34027H | 0.35 |
| A34079Hi | 0.35 |
| A34076Hi | 0.35 |
| A34065Hi | 0.36 |
| A34092Hi | 0.37 |
| A34021H | 0.37 |
| A34043Hi | 0.38 |
| A34030H | 0.38 |
| A34049Hi | 0.38 |
| A34057Hi | 0.39 |
| A34074Hi | 0.39 |
| A34048Hi | 0.39 |
| A34046Hi | 0.40 |
| A34072Hi | 0.41 |
| A34029H | 0.42 |
| A34038Hi | 0.42 |
| A34091Hi | 0.43 |
| A34023H | 0.43 |
| A34064Hi | 0.43 |
| A34104Hi | 0.44 |
| A34081Hi | 0.45 |
| A34053Hi | 0.45 |
| A34031H | 0.45 |
| A34045Hi | 0.45 |
| A34068Hi | 0.45 |
| A34071Hi | 0.47 |
| A34020H | 0.47 |
| A34036Hi | 0.47 |
| A34054Hi | 0.47 |
| A34066Hi | 0.48 |
| A34067Hi | 0.48 |
| A34086Hi | 0.49 |
| A34050Hi | 0.49 |
| A34024H | 0.49 |
| A34022H | 0.49 |
| A34032H | 0.50 |
| A34051Hi | 0.50 |
| A34098Hi | 0.51 |
| A34013H | 0.51 |
| A34007H | 0.51 |
| A34056Hi | 0.51 |
| A34070Hi | 0.51 |
| A34016H | 0.53 |
| A34094Hi | 0.54 |
| A34004H | 0.54 |
| A34085Hi | 0.56 |
| A34041Hi | 0.57 |
| A34037Hi | 0.58 |
| A34010H | 0.59 |
| A34039Hi | 0.60 |
| A34017H | 0.61 |
| A34090Hi | 0.62 |
| A34028H | 0.62 |
| A34033H | 0.63 |
| A34088Hi | 0.64 |
| A34093Hi | 0.65 |
| A34009H | 0.67 |
| A34097Hi | 0.68 |
| A34095Hi | 0.69 |
| A34002H | 0.71 |
| A34069Hi | 0.71 |
| A34008H | 0.72 |
| A34035Hi | 0.72 |
| A34102Hi | 0.73 |
| A34040Hi | 0.73 |
| A34003H | 0.73 |
| A34059Hi | 0.73 |
| A34015H | 0.74 |
| A34001H | 0.74 |
| A34099Hi | 0.75 |
| A34063Hi | 0.76 |
| A34014H | 0.77 |
| A34103Hi | 0.80 |
| A34055Hi | 0.84 |
| A34005H | 0.84 |
| A34042Hi | 0.84 |
| A34006H | 0.86 |
| Control oligo | 0.87 |
| A34087Hi | 0.87 |
| A34075Hi | 0.88 |
| A34034H | 0.88 |
| A34101Hi | 0.94 |
| A34089Hi | 1.02 |
| A34096Hi | 1.02 |

### Example 2: Determination of IC₅₀ values of selected MTDH antisense oligonucleotides in human cancer cells

The dose-dependent knockdown of MTDH mRNA expression by MTDH antisense oligonucleotides in EFO-21 cells was investigated and the respective IC₅₀ values were calculated. Therefore, EFO-21 cells were treated for three days with the respective antisense oligonucleotide at the following concentrations without the use of a transfection reagent: 6 µM, 1.5 µM, 375 nM, 94 nM, 24 nM, 6 nM, 1.5 nM. After the treatment period, cells were lysed, MTDH and HPRT1 mRNA expression was analyzed using the QuantiGene Singleplex assay (ThermoFisher) and the MTDH expression values were normalized to HPRT1 values. Residual MTDH mRNA expression as compared to mock-treated cells is depicted. A dose-dependent knockdown of MTDH with all tested antisense oligonucleotides was observed (Figure 2 and Table 6) with IC₅₀ values in the submicromolar range (Table 6). Table 6 is presented in the following:

**Table 6: Dose-dependent inhibition of MTDH mRNA expression in EFO-21 cells by selected MTDH antisense oligonucleotides and respective IC₅₀ values.**

| | | **Residual MTDH mRNA expression** | | | | | | |
|---|---|---|---|---|---|---|---|---|
| **ASO** | **IC₅₀ (nM)** | **6 µM** | **1.5 µM** | **375 nM** | **94 nM** | **24 nM** | **6 nM** | **1.5 nM** |
| A34011HM | 180.1 | 0.016 | 0.102 | 0.288 | 0.711 | 0.948 | 1.008 | 1.342 |
| A34018H | 975.2 | 0.102 | 0.391 | 0.516 | 0.705 | 0.808 | 0.904 | 1.111 |
| A34019H | 742.6 | 0.098 | 0.282 | 0.538 | 0.775 | 0.819 | 0.955 | 0.982 |
| A34025HM | 586.1 | 0.131 | 0.371 | 0.636 | 0.967 | 1.197 | 1.052 | 0.873 |
| A34027H | 375.4 | 0.134 | 0.332 | 0.558 | 0.898 | 1.240 | 1.178 | 1.062 |
| A34052Hi | 1041 | 0.169 | 0.413 | 0.915 | 1.052 | 1.381 | 1.125 | 1.005 |
| A34062Hi | 346.6 | 0.102 | 0.206 | 0.557 | 0.731 | 1.121 | 1.188 | 1.043 |
| A34077Hi | 687.3 | 0.122 | 0.343 | 0.703 | 1.008 | 1.238 | 1.101 | 1.155 |

### Example 3: Efficacy screen of MTDH antisense oligonucleotides in mouse cancer cell lines

In order to investigate the knockdown efficacy of the in silico designed MTDH antisense oligonucleotide, efficacy screening experiments were performed in two cell lines, namely Renca and 4T1. Therefore, cells were treated with the respective antisense oligonucleotide at a concentration of 5 µM for three days without the use of a transfection reagent. Cells were lysed after three days treatment period, MTDH and HPRT1 mRNA expression was analyzed using the QuantiGene Singleplex assay (ThermoFisher) and the MTDH expression values were normalized to HPRT1 values. As shown in Figure 3 A) and Table 7, treatment with 21 of 104 (20 %) antisense oligonucleotides resulted in a knockdown efficacy of > 80 % (represented by a residual MTDH mRNA expression of < 0.2 compared to mock-treated cells). Treatment with the control oligo negl had no relevant impact on MTDH mRNA expression. As shown in Figure 3 B) and Table 8, treatment with 40 of 104 (38 %) antisense oligonucleotides resulted in a knockdown efficacy of > 80 % (represented by a residual MTDH mRNA expression of < 0.2 compared to mock-treated cells). Treatment with the control oligo negl had no relevant impact on MTDH mRNA expression. Tables 7 and 8 are shown in the following:

**Table 7: List of the mean MTDH mRNA expression values in antisense oligonucleotide-treated Renca cells compared to mock-treated cells. Expression values are normalized to HPRT1.**

| **ASO** | **Residual MTDH mRNA expression (compared to mock-treated cells)** |
|---|---|
| A34073Mi | 0.06 |
| A34095Mi | 0.07 |
| A34039M | 0.09 |
| A34029HM | 0.09 |
| A34082Mi | 0.10 |
| A34050Mi | 0.10 |
| A34023M | 0.11 |
| A34030M | 0.12 |
| A34064Mi | 0.12 |
| A34036M | 0.12 |
| A34026M | 0.13 |
| A34052Mi | 0.13 |
| A34025HM | 0.13 |
| A34097Mi | 0.13 |
| A34068Mi | 0.14 |
| A34063Mi | 0.15 |
| A34051Mi | 0.16 |
| A34038M | 0.16 |
| A34055Mi | 0.18 |
| A34065Mi | 0.19 |
| A34022M | 0.19 |
| A34021M | 0.20 |
| A34084Mi | 0.21 |
| A34037M | 0.21 |
| A34035M | 0.22 |
| A34028HM | 0.22 |
| A34059Mi | 0.22 |
| A34088Mi | 0.23 |
| A34102Mi | 0.24 |
| A34087Mi | 0.25 |
| A34096Mi | 0.27 |
| A34090Mi | 0.29 |
| A34034M | 0.30 |
| A34041M | 0.32 |
| A34077Mi | 0.34 |
| A34040M | 0.35 |
| A34080Mi | 0.35 |
| A34061Mi | 0.36 |
| A34094Mi | 0.36 |
| A34101Mi | 0.36 |
| A34060Mi | 0.37 |
| A34093Mi | 0.38 |
| A34086Mi | 0.39 |
| A34010M | 0.39 |
| A34091Mi | 0.41 |
| A34103Mi | 0.44 |
| A34072Mi | 0.47 |
| A34089Mi | 0.47 |
| A34011M | 0.47 |
| A34009M | 0.47 |
| A34081Mi | 0.48 |
| A34008M | 0.48 |
| A34074Mi | 0.50 |
| A34066Mi | 0.51 |
| A34079Mi | 0.52 |
| A34057Mi | 0.53 |
| A34083Mi | 0.55 |
| A34054Mi | 0.55 |
| A34012M | 0.55 |
| A34046HM | 0.56 |
| A34031M | 0.57 |
| A34053Mi | 0.58 |
| A34024M | 0.58 |
| A34002M | 0.59 |
| A34085Mi | 0.59 |
| A34076Mi | 0.61 |
| A34003M | 0.62 |
| A34092Mi | 0.64 |
| A34067Mi | 0.67 |
| A34104Mi | 0.69 |
| A34075Mi | 0.71 |
| A34033M | 0.71 |
| A34056Mi | 0.74 |
| A34098Mi | 0.75 |
| A34027M | 0.76 |
| A34078Mi | 0.77 |
| A34020HM | 0.78 |
| A34017M | 0.78 |
| A34062Mi | 0.79 |
| A34004M | 0.79 |
| A34019M | 0.80 |
| A34032M | 0.80 |
| A34013M | 0.81 |
| A34005M | 0.81 |
| A34071Mi | 0.82 |
| A34069Mi | 0.84 |
| A34100Mi | 0.87 |
| A34042M | 0.87 |
| A34048M | 0.89 |
| A34001M | 0.90 |
| A34043M | 0.90 |
| A34006M | 0.91 |
| A34014M | 0.91 |
| A34044M | 0.92 |
| A34007M | 0.93 |
| A34058Mi | 0.99 |
| A34016M | 1.03 |
| A34018M | 1.03 |
| A34047M | 1.05 |
| A34070Mi | 1.07 |
| A34049M | 1.07 |
| A34099Mi | 1.09 |
| A34015M | 1.11 |
| A34045M | 1.15 |
| Control oligo | 1.33 |

**Table 8: List of the mean MTDH mRNA expression values in antisense oligonucleotide-treated 4T1 cells compared to mock-treated cells. Expression values are normalized to HPRT1.**

| **ASO** | **Residual MTDH mRNA expression (compared to mock-treated cells)** |
|---|---|
| A34095Mi | 0.01 |
| A34026M | 0.02 |
| A34073Mi | 0.02 |
| A34022M | 0.02 |
| A34025HM | 0.03 |
| A34023M | 0.03 |
| A34050Mi | 0.04 |
| A34082Mi | 0.04 |
| A34039M | 0.04 |
| A34052Mi | 0.04 |
| A34041M | 0.04 |
| A34087Mi | 0.04 |
| A34051Mi | 0.05 |
| A34036M | 0.05 |
| A34064Mi | 0.05 |
| A34029HM | 0.06 |
| A34021M | 0.07 |
| A34068Mi | 0.07 |
| A34055Mi | 0.07 |
| A34063Mi | 0.07 |
| A34088Mi | 0.07 |
| A34090Mi | 0.08 |
| A34038M | 0.09 |
| A34028HM | 0.10 |
| A34059Mi | 0.11 |
| A34035M | 0.11 |
| A34065Mi | 0.11 |
| A34097Mi | 0.11 |
| A34093Mi | 0.12 |
| A34084Mi | 0.13 |
| A34101Mi | 0.13 |
| A34037M | 0.13 |
| A34040M | 0.14 |
| A34102Mi | 0.14 |
| A34030M | 0.15 |
| A34089Mi | 0.15 |
| A34043M | 0.16 |
| A34103Mi | 0.17 |
| A34024M | 0.18 |
| A34096Mi | 0.18 |
| A34046HM | 0.21 |
| A34086Mi | 0.22 |
| A34061Mi | 0.23 |
| A34008M | 0.23 |
| A34060Mi | 0.23 |
| A34077Mi | 0.26 |
| A34034M | 0.27 |
| A34048M | 0.28 |
| A34011M | 0.28 |
| A34080Mi | 0.29 |
| A34074Mi | 0.34 |
| A34057Mi | 0.35 |
| A34083Mi | 0.36 |
| A34016M | 0.36 |
| A34044M | 0.37 |
| A34067Mi | 0.37 |
| A34015M | 0.37 |
| A34094Mi | 0.38 |
| A34032M | 0.42 |
| A34085Mi | 0.42 |
| A34018M | 0.43 |
| A34066Mi | 0.44 |
| A34091Mi | 0.44 |
| A34078Mi | 0.45 |
| A34069Mi | 0.48 |
| A34017M | 0.49 |
| A34076Mi | 0.50 |
| A34092Mi | 0.50 |
| A34014M | 0.52 |
| A34010M | 0.53 |
| A34020HM | 0.53 |
| A34009M | 0.54 |
| A34033M | 0.55 |
| A34072Mi | 0.57 |
| A34031M | 0.58 |
| A34012M | 0.59 |
| A34003M | 0.60 |
| A34013M | 0.61 |
| A34054Mi | 0.63 |
| A34019M | 0.64 |
| A34049M | 0.66 |
| A34079Mi | 0.67 |
| A34075Mi | 0.70 |
| A34104Mi | 0.70 |
| A34002M | 0.71 |
| A34099Mi | 0.74 |
| A34053Mi | 0.74 |
| A34056Mi | 0.76 |
| A34042M | 0.76 |
| A34058Mi | 0.77 |
| A34100Mi | 0.79 |
| A34027M | 0.80 |
| A34045M | 0.81 |
| A34070Mi | 0.84 |
| A34098Mi | 0.86 |
| A34001M | 0.87 |
| A34062Mi | 0.87 |
| A34006M | 0.94 |
| A34007M | 1.00 |
| A34071Mi | 1.06 |
| A34005M | 1.07 |
| Control oligo | 1.10 |
| A34004M | 1.14 |
| A34081Mi | 1.15 |
| A34047M | 1.16 |

### Example 4: Determination of IC₅₀ values of selected MTDH antisense oligonucleotides in mouse cancer cells

The dose-dependent knockdown of MTDH mRNA expression by MTDH antisense oligonucleotides in 4T1 cells was investigated and the respective IC₅₀ values were calculated. Therefore, 4T1 cells were treated for three days with the respective antisense oligonucleotide at the following concentrations without the use of a transfection reagent: 6 µM, 1.5 µM, 375 nM, 94 nM, 24 nM, 6 nM, 1.5 nM. After the treatment period, cells were lysed, MTDH and HPRT1 mRNA expression was analyzed using the QuantiGene Singleplex assay (ThermoFisher) and the MTDH expression values were normalized to HPRT1 values. Residual MTDH mRNA expression as compared to mock-treated cells is depicted. We observed a dose-dependent knockdown of MTDH with all tested antisense oligonucleotides (Fig. 4 and Table 9) with IC₅₀ values in the submicromolar range (Table 9). Table 9 is shown in the following:

**Table 9: Dose-dependent inhibition of MTDH mRNA expression in 4T1 cells by selected MTDH antisense oligonucleotides and respective IC₅₀ values.**

| | | **Residual MTDH mRNA expression** | | | | | | |
|---|---|---|---|---|---|---|---|---|
| **ASO** | **IC₅₀ (nM)** | **6 µM** | **1.5 µM** | **375 nM** | **94 nM** | **24 nM** | **6 nM** | **1.5 nM** |
| A34022M | 410 | 0.20 | 0.24 | 0.53 | 0.80 | 0.72 | 0.85 | 0.81 |
| A34023M | 341 | 0.21 | 0.27 | 0.61 | 1.11 | 1.11 | 1.16 | n.d. |
| A34051Mi | 164 | 0.12 | 0.24 | 0.38 | 0.55 | 0.76 | 0.94 | 0.78 |
| A34052Mi | 138 | 0.11 | 0.20 | 0.36 | 0.52 | 0.63 | 0.79 | n.d. |
| A34055Mi | 586 | 0.19 | 0.33 | 0.52 | 0.65 | 0.78 | 0.77 | 0.75 |
| A34059Mi | 239 | 0.13 | 0.24 | 0.45 | 0.67 | 0.91 | 1.02 | 0.84 |
| A34068Mi | 72 | 0.16 | 0.21 | 0.33 | 0.60 | 0.95 | 1.23 | n.d. |
| A34082Mi | 257 | 0.07 | 0.12 | 0.35 | 0.83 | 0.89 | 0.92 | n.d. |

## Claims

1. MTDH inhibitor consisting of an antisense oligonucleotide comprising 12 to 25 nucleotides, wherein at least one of the nucleotides is modified, and the oligonucleotide hybridizes with a nucleic acid sequence of MTDH of SEQ ID NO.1, of SEQ ID NO.2 or a combination thereof, wherein the oligonucleotide inhibits at least 50 % of the MTDH expression.

2. Inhibitor according to claim 1, wherein the modified nucleotide is selected from the group consisting of a bridged nucleic acid such as LNA, cET, ENA, 2'Fluoro modified nucleotide, 2'O-Methyl modified nucleotide, a 2'O-Methoxy modified nucleotide, a FANA and a combination thereof.

3. Inhibitor according to claim 1 or 2, wherein the antisense oligonucleotide hybridizes with a hybridizing active region selected from the group consisting of position 52000 to 52499, position 32000 to 32499, position 87500 to 87999, position 90500 to 90999, position 65500 to 65999, position 8500 to 8999, position 9000 to 9499, position 9500 to 9999, position 1000 to 10499, position 10500 to 10999, position 11000 to 11499, position 13000 to 13499, position 14000 to 14499, position 15500 to 15999, position 16500 to 16999, position 17500 to 17999, position 18000 to 18499, position 20500 to 20999, position 21000 to 21499, position 22500 to 22999, position 24000 to 24499, position 25000 to 25499, position 25500 to 25999, position 27000 to 27499, position 29000 to 29499, position 29500 to 29999, position 37000 to 37499, position 37500 to 37999, position 43500 to 43999, position 44500 to 44999, position 45500 to 45999, position 46500 to 46999, position 47000 to 47499, position 49000 to 49499, position 50500 to 50999, position 52500 to 52999, position 54000 to 54499, position 55500 to 55999, position 61500 to 61999, position 64000 to 64499, position 64500 to 64999, position 65000 to 65499, position 68000 to 68499, position 68500 to 68999, position 71500 to 71999, position 72000 to 72499, position 74500 to 74999, position 76000 to 76499, position 77000 to 77499, position 77500 to 77999, position 78000 to 78499, position 80500 to 80999, position 81000 to 81499, position 81500 to 81999, position 82000 to 82499, position 83500 to 83999, position 85000 to 85499, position 86000 to 86499, position 88500 to 88999, position 89000 to 89499, position 89500 to 89999, position 90000 to 90499, position 91000 to 91499, position 92000 to 92499, position 92500 to 92999, position 93500 to 93999, position 94500 to 94999 or a combination thereof.

4. The inhibitor according to any one of claims 1 to 3 comprising a sequence selected from the group consisting of SEQ ID NO.13, SEQ ID NO.64, SEQ ID NO.20, SEQ ID NO.21, SEQ ID NO.29, SEQ ID NO.27, SEQ ID NO.79, one of SEQ ID NO.3 to SEQ ID NO.12, one of SEQ ID NO.14 to SEQ ID NO.19, one of SEQ ID NO.22 to SEQ ID NO.26, SEQ ID NO.28, one of SEQ ID NO.30 to SEQ ID NO.63, one of SEQ ID NO.65 to SEQ ID NO.78, one of SEQ ID NO.80 to SEQ ID NO.221 and a combination thereof.

5. The inhibitor of any one of claims 1 to 4, wherein the antisense oligonucleotide is selected from the group consisting of +G*+T*+A*A*G*T*T*G*C*T*C*G*G*T*+G*+G*+T (A34011HM; SEQ ID NO.13),
+C*+A*+C*G*G*C*T*T*G*T*C*T*A*T*+C*+A*+G (A34062Hi; SEQ ID NO.64),
+T*+T*+G*T*A*G*T*A*T*T*G*G*C*+G*+G*+C (A34018H; SEQ ID NO.20),
+C*+T*+T*G*T*A*G*T*A*T*T*G*G*C*+G*+G*+C (A34019H; SEQ ID NO.21),
+C*+G*+C*A*A*T*A*C*T*G*T*T*G*A*+A*+C*+C (A34027H; SEQ ID NO.29),
+C*+G*+T*T*T*G*G*T*A*A*A*G*G*C*+T*+A*+T (A34025HM; SEQ ID NO.27),
+T*+C*+G*T*A*T*C*T*A*C*T*G*T*C*+T*+A*+A (A34077Hi; SEQ ID NO.79),
+C*+T*+T*A*T*C*A*C*G*T*T*T*A*C*+G*+C*+T (A34010H; SEQ ID NO.12),
+G*+A*+T*G*C*G*G*T*T*G*T*A*A*G*+T*+T*+G (A34012H; SEQ ID NO. 14),
+T*+G*+C*T*C*G*G*T*G*G*T*A*A*C*+T*+G*+T (A34113HM; SEQ ID NO.115),
+A*+A*+G*T*T*G*C*T*C*G*G*T*G*G*+T*+A*+A (A34114HM; SEQ ID NO.116),
+T*+G*+A*T*G*C*G*G*T*T*G*T*A*A*+G*+T*+T (A34115H; SEQ ID NO.117),
+A*+A*+C*A*C*T*G*C*T*G*G*T*A*T*+T*+C*+G (A34137Hi; SEQ ID NO. 139),
+A*+G*+C*T*T*C*C*T*T*T*A*A*G*C*+G*+A*+C (A34063Hi; SEQ ID NO.65),
+C*+G*+T*T*C*T*T*G*G*C*G*C*C*A*+C*+A*+T (A34026H; SEQ ID NO.28),
+C*+A*+C*G*T*T*T*G*G*T*A*A*A*G*+G*+C*+T (A34122HM; SEQ ID NO.124),
+C*+G*+C*C*A*G*C*T*T*A*C*C*T*T*+G*+A*+T (A34075Hi; SEQ ID NO.77),
+T*+G*+T*C*G*C*C*A*G*C*T*T*A*C*+C*+T*+T (A34189Hi; SEQ ID NO.191) and a combination thereof, wherein + indicates an LNA nucleotide and * indicates a phosphorothioate (PTO) linkage between the nucleotides.

6. The inhibitor of any one of claims 1 to 5, wherein the inhibitor inhibits the expression of MTDH at a nanomolar or micromolar concentration.

7. A pharmaceutical composition comprising an inhibitor of any one of claims 1 to 6 and a pharmaceutically acceptable carrier, excipient, dilutant or a combination thereof.

8. The pharmaceutical composition of claim 7, further comprising another active agent, another oligonucleotide, an antibody, a peptide-based therapeutic, a protein-based therapeutic and/or a small molecule.

9. The Inhibitor of any one of claims 1 to 6 or the pharmaceutical composition according to claim 7 or 8 for use in a method of preventing and/or treating a disorder, where an MTDH imbalance is involved.

10. The inhibitor or the pharmaceutical composition for use according to claim 9, wherein the disorder is tumor such as a malignant or benign tumor or a kidney disease.

11. The inhibitor or the pharmaceutical composition for use according to claim 10, wherein the tumor is selected from the group consisting of breast cancer, lung cancer, malignant melanoma, lymphoma, skin cancer, bone cancer, prostate cancer, liver cancer, brain cancer, cancer of the larynx, gall bladder, pancreas, testicular, rectum, parathyroid, thyroid, adrenal, neural tissue, head and neck, colon, stomach, bronchi, kidneys, basal cell carcinoma, squamous cell carcinoma, metastatic skin carcinoma, osteo sarcoma, Ewing's sarcoma, reticulum cell sarcoma, liposarcoma, myeloma, giant cell tumor, small-cell lung tumor, islet cell tumor, primary brain tumor, meningioma, acute and chronic lymphocytic and granulocytic tumors, acute and chronic myeloid leukemia, hairy-cell tumor, adenoma, hyperplasia, medullary carcinoma, intestinal ganglioneuromas, Wilm's tumor, seminoma, ovarian tumor, leiomyomater tumor, cervical dysplasia, retinoblastoma, soft tissue sarcoma, malignant carcinoid, topical skin lesion, rhabdomyosarcoma, Kaposi's sarcoma, osteogenic sarcoma, malignant hypercalcemia, renal cell tumor, polycythermia vera, adenocarcinoma, anaplastic astrocytoma, glioblastoma multiforma, leukemia, epidermoid carcinoma and a kidney disease such as diabetic nephropathy.

12. The inhibitor or the pharmaceutical composition for use according to any one of claims 9 or 11, wherein the inhibitor or the composition is suitable to be administered locally or systemically.
